# EUROPEAN PATENT APPLICATION

(11) **EP 4 332 874 A1**
(43) Date of publication of application: **06.03.2024**
(21) Application number: 22853331.1
(22) Date of filing: 27.07.2022
(51) Int. Cl.: G06Q 50/22, G06Q 50/10, H04M 1/72448, G06F 3/0482, G06F 3/14

(54) **ELECTRONIC DEVICE FOR GENERATING SPORT, AND OPERATING METHOD OF ELECTRONIC DEVICE**

(30) Priority: 02.08.2021 KR 20210101581
(71) Applicant: Samsung Electronics Co., Ltd., Suwon-si, Gyeonggi-do 16677 (KR)
(72) Inventor: LEE, Eunhye, Suwon-si Gyeonggi-do 16677 (KR); LIM, Cheolkyu, Suwon-si Gyeonggi-do 16677 (KR)
(74) Representative: Appleyard Lees IP LLP
(86) International application number: PCT/KR2022/011001
(87) International publication number: WO 2023/013953

(57) **Abstract**

The present document relates to an electronic device for generating a sport, and an operating method of the electronic device, and according to an embodiment, the electronic device comprises a memory and at least one processor connected to the memory, wherein the at least one processor may be configured to: obtain a sport name in response to a sport addition request; recommend at least one data type on the basis of the sport name; generate a new sport corresponding to the sport name on the basis of a data type selected from the recommended at least one data type; and obtain sports measurement information corresponding to the selected data type in response to execution of the new sport. Other embodiments are also possible.

## Description

### [Technical Field]

The disclosure relates to an electronic device for generating a new workout type through a workout function and a method of operating the electronic device.

### [Background Art]

Recently, electronic devices have been developed in various forms for convenience of users and provide a variety of services or functions.

A variety of services or functions of electronic devices include a workout function for increasing health of users. When providing the workout function, the electronic device may provide workout guides of various workout types and a workout result according to execution of respective workout types in order to efficiently conduct workouts.

### [Detailed Description of the Invention]

### [Technical Problem]

An electronic device providing a workout function may provide various workout types through the workout function but cannot provide all of the existing workout types. Every users prefer different workout types and different numbers of workout types which can be provided may be configured according to characteristics of electronic devices (for example, structure, memory capacity, and the like), and thus the electronic device may have difficulty in providing workout types in consideration of characteristics of the electronic device or user preference. Since an electronic device such as a wearable device having small capacity may provide the smaller number of workout types in compared to other electronic devices having large capacity, it may be difficult to provide a workout type which the user desires because, when the wearable device performs the workout function through interworking with other electronic devices, provided workout types do not match.

According to an embodiment of the disclosure, an electronic device and a method of operating the electronic device for generating new workout types to provide various workout types through the workout function may be provided.

### [Technical Solution]

According to an embodiment of the disclosure, an electronic device includes a memory and at least one processor connected to the memory.

According to an embodiment, the at least one processor is configured to acquire a workout type name in response to a request for adding a workout type.

According to an embodiment, the at least one processor is configured to recommend at least one data type, based on the workout type name.

According to an embodiment, the at least one processor is configured to generate a new workout type corresponding to the workout type name, based on a data type selected from among the at least one recommended data type.

According to an embodiment, the at least one processor is configured to acquire workout measurement information corresponding to the selected data type in response to execution of the new workout type.

According to an embodiment, a method of operating an electronic device includes acquiring a workout type name in response to a request for adding a workout type.

According to an embodiment, a method of operating an electronic device includes recommending at least one data type, based on the workout type name.

According to an embodiment, a method of operating an electronic device includes generating a new workout type corresponding to the workout type name, based on a data type selected from among the at least one recommended data type.

According to an embodiment, a method of operating an electronic device includes acquiring workout measurement information corresponding to the selected data type in response to execution of the new workout type.

### [Advantageous Effects]

According to an embodiment, an electronic device and a method of operating the electronic device can conveniently and efficiently provide various new workout types in consideration of a characteristic of the electronic device and user preference.

Further, according to an embodiment, an electronic device and a method of operating the electronic device can conveniently add a new workout type to be executed through synchronization with another electronic device although workout types are different when the electronic device such as a wearable device operates on the basis of an interworking with the other electronic device, thereby providing more various workout types compared to a device providing a limited number of workout types due to a memory capacity or the like.

### [Brief Description of Drawings]

FIG. 1 is a block diagram illustrating an electronic device within a network environment according to various embodiments.
FIG. 2 is a block diagram illustrating an example of a configuration of the second electronic device according to an embodiment.
FIG. 3 illustrates an example of a configuration of the first electronic device and the second electronic device for generating workout types according to an embodiment.
FIGs. 4A and 4B illustrate an example in which the first electronic device and the second electronic device generate workout types according to an embodiment.
FIG. 5 illustrates an example of an operation method of electronic devices according to an embodiment.
FIGs. 6A and 6B illustrate an example in which the second electronic device generates workout types according to an embodiment.
FIGs. 7A, 7B, and 7C illustrate an example in which the first electronic device generates workout types according to an embodiment.
FIG. 8A illustrates an example of an execution result of a new workout type in the second electronic device according to an embodiment,
FIG. 8B illustrates an example of an execution result of a new workout type in the first electronic device according to an embodiment.
FIG. 9 illustrates an example of a workout measurement result and an example of an execution result of a new workout type in the first electronic device according to an embodiment.
FIGs. 10A and 10B illustrate an example of an execution result of a new workout type in the second electronic device according to an embodiment.
FIGs. 11A, 11B, 11C, and 11D illustrate an example of an execution result of a new workout type in the first electronic device according to an embodiment.
FIG. 12 illustrates an example of an execution result of a new workout type in the second electronic device according to an embodiment.
FIG. 13 illustrates an example of an execution result of a new workout type in the first electronic device according to an embodiment.
FIG. 14 illustrates an example of a method of operating electronic devices according to an embodiment.

In connection with description of drawings, the same or similar reference numerals may be used for the same or similar elements.

### [Mode for Carrying out the Invention]

Hereinafter, an electronic device according to various embodiments is described with reference to the accompanying drawings. The term user used in various embodiments may be a person using an electronic device or a device (for example, an artificial intelligence electronic device) using an electronic device.

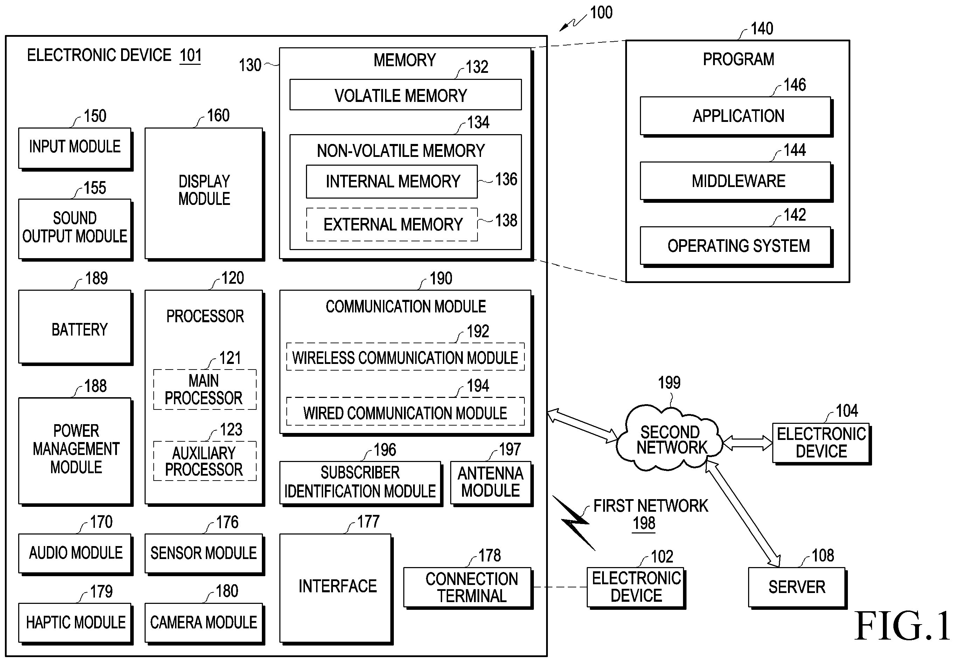

FIG. 1 is a block diagram illustrating an electronic device 101 in a network environment 100 according to various embodiments. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control, for example, at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active (e.g., executing an application) state. According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence model is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or an external electronic device (e.g., an electronic device 102 (e.g., a speaker or a headphone)) directly or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device 104 via the first network 198 (e.g., a short-range communication network, such as BluetoothTM, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20 Gbps or more) for implementing 1eMBB, loss coverage (e.g., 164 dB or less) for implementing mMTC, or U-plane latency (e.g., 0.5 ms or less for each of downlink (DL) and uplink (UL), or a round trip of 1 ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the external electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

Referring to FIG. 1, an electronic device 101 according to an embodiment may implement a software module (for example, a program 140 of FIG. 1) for performing a workout function or adding a new workout type. A memory 130 of the electronic device 101 may store instructions for implementing a software module. At least one processor 120 may execute the instructions stored in the memory 130 in order to implement the software module and may control hardware (for example, a sensor module 176, a power management module 188, or a communication module 190 of FIG. 1) related to a function of the software module. At least a portion of the software module may be implemented by software, firmware, hardware, or a combination of two or more thereof. At least a portion of the software module may be implemented (for example, executed) by, for example, a processor (for example, AP). At least a part of the software module may include, for example, a module, a program, a routine, a set of instructions, or a process for performing at least one function.

According to an embodiment, the processor 120 of the electronic device 101 may identify an input workout type name in response to a request for adding a workout type. For example, the processor 120 may identify a workout type name input by a user's utterance, a text input, or selection of one of recommended workout types displayed on a screen. For example, the processor 120 may receive, from an external electronic device (for example, an electronic device 102 or 104 of FIG. 1), a workout type name of a workout type to be added.

According to an embodiment, the processor 120 may recommend at least one data type on the basis of a workout type name. The processor 120 may store in advance mapping information between workout type names and at least one data type in the memory 130. The processor 120 may search for an input workout type name through an external electronic device (for example, the electronic device 102 or 104 or a server 104 of FIG. 1) or the Internet and acquire at least one piece of information on a similar workout type name, a relevant workout type, or a relevant data type. For example, the processor 120 may compare the input workout type name with preset workout type names included in mapping information, determine a workout type name having the highest similarity, and select the workout type name having the highest similarity as the recommended data type. The processor 120 may determine a data type having a high correlation with the input workout type name based on information acquired from the outside among at least one data type as the recommended data type. The processor 120 may determine a data type having a high correlation with the input workout type name through an artificial intelligence module within the first electronic device 101 as the recommended data type. The processor 120 may recommend a data type on the basis of recent workout measurement information of the user as well as the similarity or the correlation with the input workout type name. Further, the processor 120 may display and recommend a plurality of data types having the correlation according to a correlation priority.

According to an embodiment, the processor 120 may select a data type for acquiring workout measurement information from among at least one recommended data type. Each data type corresponds to a preset workout type and may include one or more measurement elements for acquiring workout measurement information suitable for the preset workout type. That is, the data type may include a group of one or more measurement elements for acquiring an amount of the workout and biosensing data according to execution of the workout by the user in accordance with a workout type corresponding to the corresponding data type. At least one data type may include an indoor workout, outdoor running, circuit training, or the like. Each data type may include at least one measurement element among a distance, a speed, pace, calories, a heart rate, workout measurement duration, the number of repetitive workouts, cadence, or a workout set. In addition, the data type may further include another element which can be measured as the measurement element when the workout type is executed. Each measurement element may be information which can be acquired on the basis of sensing data received from at least one sensor, a sensor module, or an external device. When the data type or the measurement element is designated, the electronic device may acquire measurement data corresponding to the designated data type or measurement element through at least one sensor, the sensor module, or the external device, so as to acquire workout measurement information.

According to an embodiment, the processor 120 may generate a new workout type for acquiring workout measurement information corresponding to the selected data type and store information related to the generated new workout type. Information related to the generated new workout type may include identification information of the workout type (for example, a type name or an identification code), an icon (for example, an image or a graphic element) indicating the workout type), information on the selected data type, or additional information (for example, a priority or memo information of the user). The processor 120 may add objects (for example, at least one of an image, an icon, a graphic element, or text) indicating the generated new workout type to a workout type list which can be provided and control the display module 160 to display the workout type list.

According to an embodiment, when the generated new workout type is executed, the processor 120 may acquire measurement data measured by at least one sensor included in the sensor module 220 or a GPS module (not shown) for each of at least one measurement element designated to the selected data type. The processor 120 may acquire workout measurement information configured on the basis of measurement data measured for each of at least one measurement element and control the display module 160 to display the acquired workout measurement information.

According to an embodiment, the processor 120 may transmit information related to the new workout type, workout measurement information of the new workout type, or additional information (for example, at least one piece of weather information, user information, previous workout result information, workout measurement information of another workout type, or user input information) to the first electronic device 101 or another external electronic device through the communication module 230. The processor 120 may perform synchronization between first electronic devices 101, synchronize and perform the operation of a workout application executed in the second electronic device 201 and the operation of a workout application executed in the first electronic device 101, measure workout measurement information for a new workout type through synchronization, and share the workout measurement information.

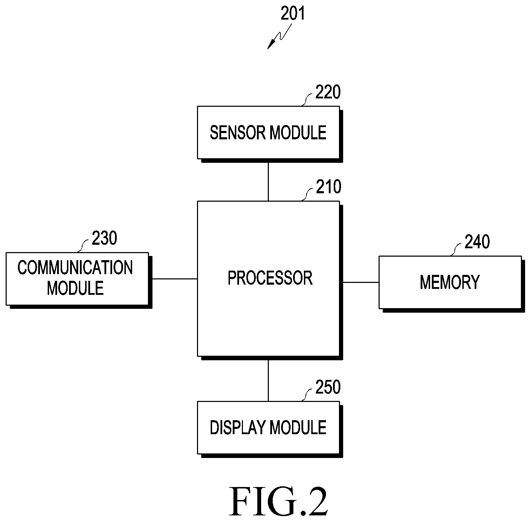

FIG. 2 is a block diagram illustrating an example of a configuration of a second electronic device according to an embodiment.

Referring to FIG. 2, a second electronic device 201 (for example, the electronic device 102 or 104 of FIG. 1) according to an embodiment may include a processor (e.g., including processing circuitry) 210, a sensor module (e.g., including at least one sensor) 220, a communication module (e.g., including communication circuitry) 230, a memory 240, and a display module (e.g., including at least one display) 250. In addition, the second electronic device 201 may further include another element required for executing a workout function or generating a new workout type. The second electronic device may be connected to a first electronic device (for example, the electronic device 101 of FIG. 1) through the communication module 230 and may be a wearable device which can be carried by or worn on the user. In another example, the electronic device 201 may be a small device which can be put on one hand of the user and can be controlled by the other hand.

According to an embodiment, the processor 210 may be electrically connected to the sensor module 220, the communication module 230, the memory 240, and the display module 250. The processor 210 may include various processing circuitry and identify an input workout type name in response to a request for adding the workout type. For example, the processor 210 may identify a workout type name input by a user's utterance, a text input, or selection of one of recommended workout types displayed on a screen. For example, the processor 210 may receive, from an external electronic device (for example, the first electronic device 101 of FIG. 1), a workout type name of the workout type to be added.

According to an embodiment, the processor 210 may recommend at least one data type on the basis of a workout type name. The processor 210 may search for a workout type name input through an external electronic device (for example, the electronic device 101 or the server 104 of FIG. 1) or the Internet and acquire at least one piece of information on a similar workout type name, a relevant workout type, or a relevant data type. For example, the processor 210 may compare the input workout type name with preset workout type names included in mapping information, determine a workout type name having the highest similarity, and select the workout type name having the highest similarity as the recommended data type. The processor 210 may determine a data type having a high correlation with the input workout type name among at least one data type as the recommended data type on the basis of information acquired from the outside. The processor 210 may determine a data type having a high correlation with the input workout type name through an artificial intelligence module within the first electronic device 201 as the recommended data type. The processor 210 may recommend a data type on the basis of recent workout measurement information of the user as well as the similarity or the correlation with the input workout type name. Further, the processor 210 may display and recommend a plurality of data types having the correlation according to a correlation priority.

According to an embodiment, the processor 210 may select a data type for acquiring workout measurement information from among at least one recommended data type. Each data type corresponds to a preset workout type and may include one or more measurement elements for acquiring workout measurement information suitable for the preset workout type. That is, the data type may include a group of one or more measurement elements for acquiring an amount of the workout and biosensing data according to execution of the workout by the user in accordance with a workout type corresponding to the corresponding data type. The data type may include at least one of a distance, a speed, pace, calories, a heart rate, workout measurement duration, the number of repetitive workouts, cadence, or a workout set as the measurement element. In addition, the data type may further include another element which can be measured as the measurement element when the workout type is executed. Each measurement element may be information which can be acquired on the basis of sensing data received from at least one sensor, a sensor module, or an external electronic device (for example, the electronic device 101 of FIG. 1). When the data type or the measurement element is designated, the electronic device may acquire measurement data corresponding to the designated data type or measurement element through at least one of at least one sensor, the sensor module, or the external electronic device, so as to acquire workout measurement information.

According to an embodiment, the processor 210 may generate a new workout type for acquiring workout measurement information corresponding to the selected data type, add the generated new workout type as a workout type which can be provided, and store information related to the new workout type in the memory 240. The information related to the generated new workout type may include identification information (for example, a type name or an identification code) of the workout type, an image (or an icon) indicating the workout type, information on a selected data type, or additional information (for example, a priority or memo information of the user). The processor 210 may add objects (for example, at least one of an image, an icon, a graphic element, or text) indicating the generated new workout type to a workout type list which can be provided and control the display module 250 to display the workout type list.

According to an embodiment, when the generated new workout type is executed, the processor 210 may acquire measurement data measured by at least one sensor included in the sensor module 220 or a GPS module (not shown) for each of at least one measurement element designated to the selected data type. The processor 210 may acquire workout measurement information configured on the basis of measurement data measured for each of at least one measurement element and control the display module 250 to display the acquired workout measurement information.

According to an embodiment, the processor 210 may transmit information related to a new workout type, workout measurement information of a new workout type, or additional information (for example, at least one piece of weather information, user information, previous workout result information, or user input information) to the first electronic device 101 or another external electronic device through the communication module 230. The processor 120 may perform synchronization between first electronic devices 101, synchronize and perform the operation of a workout application executed in the second electronic device 201 and the operation of a workout application executed in the first electronic device 101, measure workout measurement information for a new workout type through synchronization, and share the workout measurement information.

According to an embodiment, the processor 210 is a hardware module or a software module (for example, an application program) and may be a hardware component (function) or a software component (program) including at least one of various sensors included in the electronic device 201, a data measurement module, an input/output interface, a module for managing a state or an environment of the electronic device 201, or the communication module 230.

According to an embodiment, the processor 210 may include, for example, a combination of one or more of hardware, software, or firmware. At least some of the elements of the processor 210 may be omitted, or another element for performing an image processing operation as well as the elements may be further included.

According to an embodiment, the sensor module 220 may be electrically connected to the processor 210, and may include at least one sensor for measuring a workout result when the user executes the selected workout type. For example, at least one sensor may be a sensor capable of measuring at least one of a distance, a speed, pace, calories, biometric information (for example, a heart rate), workout hours, or cadence. For example, the sensor module 220 may include a global positioning system (GPS) module (not shown) for measuring the location of the second electronic device. However, the GPS module is not limited thereto, but may be included in the second electronic device 201 as a separate element.

According to an embodiment, the communication module 230 may be electrically connected to the processor 210, and may include various communication circuitry configured to perform wireless or wired communication with the first electronic device 101 or another electronic device. For example, the communication module 230 may transmit or receive information related to the generation of a new workout type and workout execution to or from the first electronic device 101 through short-range wireless communication (for example, Bluetooth communication). The communication module 260 may include a cellular module, a Wi-Fi (wireless-fidelity) module, a Bluetooth module, or a near field communication (NFC) module.

According to an embodiment, the memory 240 may be electrically connected to the processor 210 and may store various applications required for the operation. For example, the memory 130 may store applications (functions or programs) related to the workout. The memory 240 may store information related to a new workout type, workout measurement information of a new workout type, or additional information (for example, at least one piece of weather information, user information, previous workout result information, or user input information). The memory 240 may store information related to another executed workout type and workout measurement information of another workout type. The memory 240 may store in advance mapping information between workout type names and at least one data type.

According to an embodiment, the memory 240 may store various pieces of data generated during execution of the program including a program (for example, the program 140 of FIG. 1) used for operating the function. The memory 240 may largely include a program area and a data area (not shown). The program area may store program information related to driving of the electronic device 201 such as an operating system (OS) that boots the electronic device 201. The data area (not shown) may store transmitted and/or received data and generated data according to various embodiments. Further, the memory 240 may include at least one storage medium of a flash memory, a hard disk, a multimedia card micro type memory (for example, a secure digital (SD) or an extreme digital (XD) memory), a RAM, and a ROM. According to an embodiment, the memory 240 may store information related to workout execution, information related to workout types which can be provided (for example, a workout type list), information related to a new workout type, workout measurement information, and/or additional information.

According to an embodiment, the display module 250 may be electrically connected to the processor 210 and may include at least one display implemented in the form of a touch screen. When the display module 250 and an input unit are implemented in the form of a touch screen, the display module 250 may display various pieces of information generated according to a touch action of the user. The display module 250 according to an embodiment may be configured by one or more of a liquid crystal display (LCD), a thin film transistor LCD (TFT-LCD), an organic light emitting diode (OLED), LED, active matrix OLED (AMOLED), a flexible display, and a three-dimensional display. Among the displays, some displays may be configured in a transparent type or a light penetration type to look outside therethrough. The display may be implemented in a transparent display type including transparent OLED (TOLED). In addition to the display module 250, another display module (for example, an expanded display or a flexible display) may be further included.

According to an embodiment, the electronic device 201 may further include an audio module including various audio circuitry (not shown) and an input module including various input circuitry (not shown). The audio module may output a sound and may include, for example, at least one of an audio codec, a microphone (MIC), a receiver, an earphone output (EAR_L), or a speaker. The audio module may output workout result information and guidance information related to addition of a new workout type. The input module may receive input information (for example, a type name and a control command) related to addition of the new workout type by a user's utterance.

Referring to FIG. 2, the electronic device 201 according to an embodiment may implement a software module (for example, the program 140 of FIG. 1) for an operation of executing a working application or adding a new workout type. The memory 240 of the electronic device 201 may store instructions to implement the software module 201.

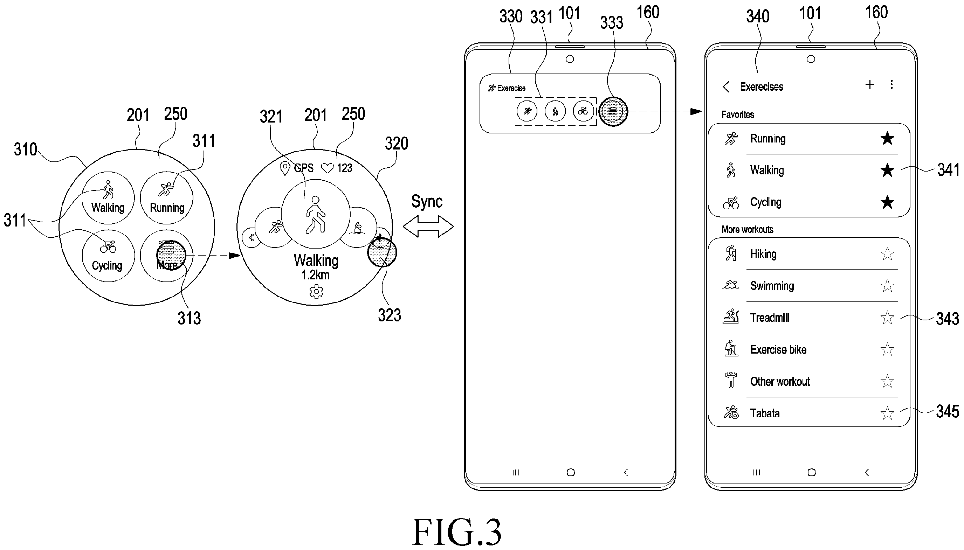

FIG. 3 illustrates an example in which the first electronic device and the second electronic device generate workout types according to an embodiment.

Referring to FIGs. 1, 2, and 3, the first electronic device 101 and the second electronic device 201 according to an embodiment may be synchronized according to execution of workout applications and may display workout types which can be provided. For example, the workout types which can be provided may be displayed in a list form.

According to an embodiment, when a workout application is executed, the processor 210 of the second electronic device 201 may control the display module 250 to display an execution screen (or widget) 310 including objects 311 (for example, images, icons, or graphic elements) indicating main workout types among the workout types which can be provided.

According to an embodiment, when an input for an object 313 indicating see more is identified on an execution screen 310, the processor 210 may control the display module 250 to display objects 321 (for example, images, icons, or graphic elements) indicating workout types which can be provided (for example, running, walking, and cycling) on the execution screen 320. The workout types 321, which can be provided, displayed on the execution screen 320 may include main workout types displayed on the execution screen 310 and other workout types which can be provided. The processor 210 may control the display module 250 to preferentially display the most recently executed workout type, the most frequently executed workout type, or a workout type having the highest priority in a portion of the display area (for example, a center area). When the number of executed main workout types is plural, the processor 210 may change the display location to display one of the workout types in the portion of the display area (for example, the center area) of the display module 250 in response to an input 323 on the execution screen 320 by a predetermined gesture (for example, swipe, tap, or scroll). The processor 210 may display such that an object indicating a workout type (for example, walking) preferentially displayed among one or more main workout types is highlighted (for example, the workout type is distinguished from other workout types by the image size, color, or visual effect). The processor 210 may control the display module 250 to display workout measurement information (for example, distance measurement data (1.2 km), heart rate measurement data (123 bpm), and location data (GPS information)) measured in accordance with a type name (for example, walking) of the workout type (for example, walking) displayed in a predetermined area (for example, a center area, an uppermost area, or a highest layer) among the work types and a predetermined data type in some areas of the execution screen 320. The workout measurement information may include measurement data measured on the basis of measurement elements included in the predetermined data type.

According to an embodiment, when a workout application is executed, the processor 120 of the first electronic device 101 may control the display module 160 to display an execution screen (or widget) 330 including objects 331 (for example, images, icons, or graphic elements) indicating main workout types among the workout types which can be provided. When an input for an object 333 indicating see more is identified on the execution screen 330, the processor 120 may control the display module 160 to display objects 341 (for example, icons and/or text) indicating main workout types (for example, running, walking, and cycling) and objects (for example, icons and/or text) 343 indicating other workout types (for example, hiking, swimming, treadmill, exercise bike, and other workout) which can be provided on the execution screen 340. For example, the processor 120 may control the display module 160 to display a list 345 including the object 341 indicating the main workout types and object 343 indicating other workout types which can be provided on the execution screen 340. The main workout types may be workout types selected by the user among the workout types which can be provided or workout types selected in an order of the larger number of executions for a recent predetermined period (for example, one week or one month). The processor 120 may display the main workout types to be distinguished from other workout types on the execution screen 340 (for example, display a selection item and/or a separate list).

According to an embodiment, the processor 210 of the second electronic device 201 may be synchronized with the operation of the workout application executed in the first electronic device 101 and transmit information related to workout types which can be provided to be executed to the first electronic device 101. When the operation of the workout application is synchronized with the operation of the workout application executed in the first electronic device 101, the execution screen 310 may be displayed while being synchronized with the execution screen 330 displayed in the first electronic device 101, and the execution screen 320 may be displayed while being synchronized with the execution screen 340 displayed in the first electronic device 101.

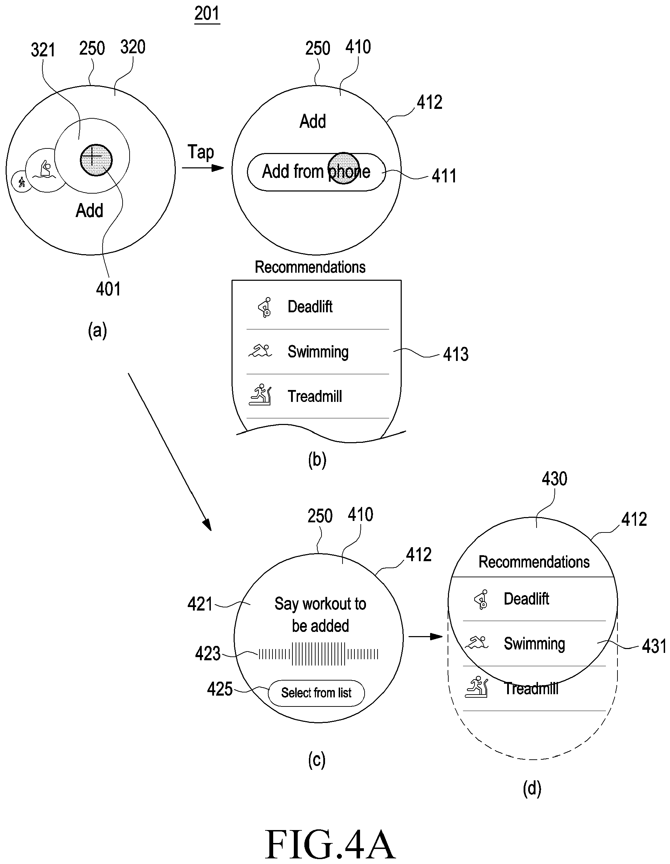

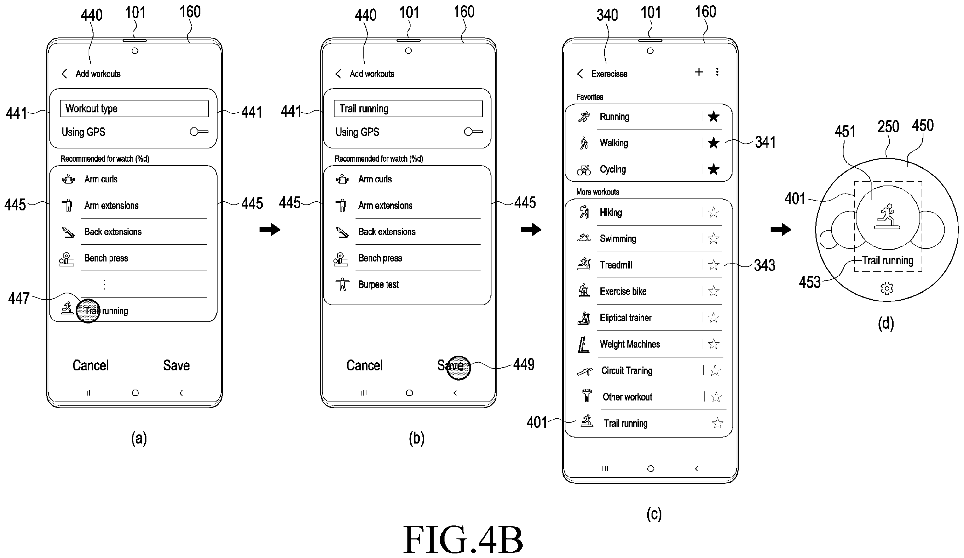

FIGs. 4A and 4B illustrate an example for adding workout types of electronic devices according to an embodiment.

Referring to FIGs. 1, 2, 3, 4A, and 4B, the processor 210 of the second electronic device 201 according to an embodiment may identify an input (for example, tap) of an addition object 401 for adding workout types displayed on the execution screen 320 as illustrated in FIG. 4A(a). The processor 210 may control the display module 250 to display an execution screen 410 for adding new workout types as illustrated in FIG. 4A(b) and an execution screen 420 for adding new workout types as illustrated in FIG. 4A(c). As illustrated in FIG. 4A(b), the processor 210 may control the display module 250 to display an object 411 (for example, add form phone) making a request for adding new workout types in the first electronic device 101 and/or a recommended list 413 including one or more recommended workout types (recommendations) (for example, at least one of deadlift, swimming, or treadmill) to add new workout types directly by the second electronic device 201 on the execution screen 410. For example, the recommended list 413 may be moved to and displayed in a display area 412 according to a predetermined gesture (for example, swipe or scroll).

According to an embodiment, when voice information by a user's utterance is input through an input module (not shown) of the second electronic device 201, the processor 210 may control the display module 250 to display an object 421 for inducing a voice input (for example, "Say a workout to be added"), an object 423 indicating detection of the voice input, and/or an object 425 for selecting a new workout type on the execution screen 420 as illustrated in FIG. 4A(c). When an input of the object 425 for selecting a new workout type (for example, "select from list") is identified on the execution screen 420, the processor 210 may control the display module 250 to display the recommended list 431 including the recommended workout types (deadlift, swimming, and treadmill) on the execution screen 430 as illustrated in FIG. 4A(d). For example, the recommended list 431 may be moved to and displayed in the display area 421 of the display module 250 on the execution screen 420 according to a predetermined gesture (for example, swipe or scroll). The display area 421 of the display module 250 is an area through which all or at least a portion of the screen of the display module 250 are exposed to the outside and may be an area for visually providing information to the outside (for example, the user).

When receiving a request for adding new workout types from the second electronic device 201 through the communication module 190, the processor 120 of the first electronic device 101 according to an embodiment may control the display module 250 to display an execution screen 440 for receiving new workout types as illustrated in FIG. 4B(a). When the request for adding the new workout types input through the input module 150 directly by the first electronic device 101 is identified, the processor 120 may control the display module 250 to display the execution screen 440 for receiving the new workout types as illustrated in FIG. 4B(a).

According to an embodiment, the processor 120 may control the display module 160 to display an object 441 indicating an input window for inputting type names of new workout types to be added and/or a recommended list 445 including recommended workout types on the execution screen 440. For example, when one of the recommended workout types (for example, arm curls, arm extensions, back extensions, bench press, and burpee test) is selected from the recommended list 445 displayed on the execution screen 440 as illustrated in FIG. 4B(a), the processor 120 may input a type name (for example, trail running) of a selected workout type 447 (for example, trail running) into the object 441 of the execution screen 440. For example, the processor 120 may identify input text corresponding to the type name from the user through the object 441 of the execution screen 440. When a save object 449 (for example, an icon, a menu, or a button) is input to generate the new workout type having the input type name, the processor 120 may add a generated new workout type (trail running) 401 to the workout type list 343 as illustrated in FIG. 4B(c). For example, the processor 120 may display an image (for example, an icon or a graphic element) indicating the new workout type 401 and text indicating the type name in the workout type list 343 of the execution screen 340. For example, when a plurality of workout types among the recommended workout types are selected from the recommended list 445 displayed on the execution screen 440, the processor 120 may display all of the type names of the selected workout types in the object 441 indicating the input window and generate new workout types for the displayed type names. For example, when a plurality of workout types among the recommended workout types are selected from the recommended list 445 displayed on the execution screen 440, the processor 120 may sequentially repeatedly perform an operation of generating new workout types for the plurality of selected workout types.

According to an embodiment, when a type name is input, the processor 120 of the first electronic device 101 may recommend at least one data type corresponding to the input type name and control the display module 160 to display an object for selecting at least one recommended data type on an execution screen (for example, the execution screen 440 of FIG. 4B). The processor 120 may control the display module 160 to display guidance information for data to be measured on the basis of at least one measurement element included in at least one data type and an object for selecting at least one recommended data type on an execution screen (for example, the execution screen 440 of FIG. 4B). The processor 120 may control the display module 160 to display an object for selecting an addition option (for example, at least one of important data or exercise intensity) for at least one recommended data type on an execution screen (for example, the execution screen 440 of FIG. 4) for adding the workout types. When a data type is selected from at least one recommended data type, the processor 120 may generate the new workout type 401 (for example, trail running) on the basis of the selected data type.

According to an embodiment, the processor 210 of the second electronic device 201 may receive information related to the new workout type generated in the first electronic device 101 from the first electronic device 101, add the new workout type 401 on the basis of the received information related to the new workout type, and control the display module 250 to display an object 451 (for example, an image, an icon, or a graphic element) indicating the new workout type 401 and the type name 453 (for example, trail running) of the new workout type 401 on the execution screen 450 as illustrated in FIG. 4B(d).

According to an embodiment, when one of the recommended workout types 413 or 431 is selected or the second electronic device 201 directly inputs the workout type, the processor 210 of the second electronic device 201 may recommend a type name corresponding to the selected workout type or at least one data type corresponding to the input type name and control the display module 250 to display an object for selecting at least one recommended data type on an execution screen (not shown) for adding the workout type as illustrated in FIGs. 4A(b) and 4A(d). For example, the processor 210 may control the display module 250 to display objects for selecting at least one recommended data type and guidance information for data to be measured in some areas of the execution screen 410 as illustrated in FIG. 4A(b) or some areas of the execution screen 250 as illustrated in FIG. 4A(d). For example, when one of the recommended workout types 413 or 431 is selected, the processor may control the display module 250 to display objects for selecting at least one recommended data type and guidance information for data to be measured by expanding an area in which the selected recommended workout type is displayed. The processor 210 may control the display module 250 to display an object for selecting an addition option (for example, at least one of important data or exercise intensity) for at least one recommended data type in some areas of the execution screen 410 as illustrated in FIG. 4A(b) or some areas of the execution screen 250 as illustrated in FIG. 4A(d). The processor 210 may generate a new workout type on the basis of a data type selected from at least one recommended data type. The processor 210 may add the generated new workout type to the workout type list which can be provided and store information related to the added new workout in the memory 240. The processor 210 may transmit information related to the new workout type to the first electronic device 101 through the communication module 240 and perform synchronization.

According to an embodiment, when the generated new workout type 401 is executed, the processor120 of the first electronic device 101 may acquire measurement data according to performance of an exercise by the user for the new workout type on the basis of at least one measurement element included in the data type of the new workout type 401. For example, all or at least some of the measurement data may be acquired through at least one sensor included in the sensor module 176 of the first electronic device 101 and/or a GPS module (not shown). In another example, all or at least some of the measurement data is data measured by at least one sensor of the second electronic device 201 or/and a GPS module and may be received from the second electronic device 201. The processor 120 may control the display module 120 to display workout measurement information including measurement data acquired on the basis of at least one measurement element. The processor 120 may be synchronized with the second electronic device 201 and may transmit workout measurement information to the second electronic device 201 to display workout measurement information. The processor 120 may control the display module 160 to display workout result information for the workout performed for a predetermined period on the basis of workout measurement information.

According to an embodiment, when the generated new workout type 401 is executed, the processor120 of the second electronic device 201 may acquire measurement data according to performance of the workout by the user for the new workout type on the basis of at least one measurement element included in the data type of the new workout type 401. For example, all or at least some of the measurement data may be acquired through at least one sensor included in the sensor module 220 of the second electronic device 201 and/or a GPS module (not shown). In another example, all or at least some of the measurement data may be received from the electronic device 101. The processor 210 may control the display module 250 to display workout measurement information including measurement data acquired on the basis of at least one measurement element. The processor 210 may be synchronized with the first electronic device 101 and may transmit workout measurement information to the first electronic device 101 to display workout measurement information. The processor 210 may control the display module 250 to display workout result information for the workout performed for a predetermined period on the basis of workout measurement information.

As described above, in an embodiment, the main elements of the electronic device have been described through the electronic device 101 of FIG. 1 and the electronic device 201 of FIG. 2. However, in various embodiments, all the elements illustrated through FIGs. 1 and 2 are not necessary elements, but the first electronic device 101 and the second electronic device 201 may be implemented by elements larger than the illustrated elements or elements fewer than the illustrated elements. Further, locations of the main elements of the first electronic device 101 and the second electronic device 201 described through FIGs. 1 and 2 may be changed according to various embodiments.

According to an embodiment, an electronic device (for example, the electronic device 101 of FIGs. 1 and 2 and the electronic device 201 of FIG. 2) may include a memory (for example, the memory 130 of the electronic device 101 of FIG. 1 or the memory 240 of the electronic device 201 of FIG. 2) and at least one processor (for example, the processor 120 of the electronic device 101 of FIG. 1 or the processor 210 of the electronic device 201 of FIG. 2) connected to the memory, and the at least one processor may be configured to acquire a workout type name in response to a request for adding a workout type, recommend at least one data type, based on the workout type name, generate a new workout type corresponding to the workout type name, based on a data type selected from among the at least one recommended data type, and acquire workout measurement information corresponding to the selected data type in response to execution of the new workout type.

According to an embodiment, the electronic device may further include a display module (for example, the display module 160 of the electronic device 101 of FIG. 1 or the display module 250 of the electronic device 201 of FIG. 2) including a display connected to the at least one processor, and the at least one processor may be configured to control the display module to display the acquired workout measurement information.

According to an embodiment, the electronic device may further include a communication module (for example, the communication module 190 of the electronic device 101 of FIG. 1 or the memory 230 of the electronic device 201 of FIG. 2) including communication circuitry connected to the at least one processor, and the at least one processor may be configured to control the communication module to transmit information related to the new workout type including the workout type name and the selected data type to an external electronic device, and wherein the communication module is configured to communicate with the external electronic device through short-range wireless communication.

According to an embodiment, the at least one processor may be configured to control the display module to display a list including recommended workout types in response to the request for adding the workout type and control the communication module to transmit the list including the recommended workout types to the external electronic device.

According to an embodiment, the electronic device may further include a sensor module comprising at least one sensor connected to the at least one processor, and the at least one processor may be configured to acquire measurement data corresponding to at least one measurement element included in the selected data type through at least one sensor included in the sensor module and acquire the workout measurement information, based on the acquired measurement data.

According to an embodiment, based on measurement data corresponding to at least one measurement element included in the selected data type being acquired from a synchronized external electronic device, the at least one processor may be configured to add the acquired measurement data from the external device and acquire the workout measurement information.

According to an embodiment, the at least one processor may be configured to perform synchronization with an external electronic device, acquire measured measurement data corresponding to at least one measurement element included in the selected data type from the external electronic device, and acquire the workout measurement information, based on the acquired measurement data.

According to an embodiment, the at least one processor may be configured to provide additional option information for the selected data type, select the provided additional option information, based on a specified priority, and add the selected additional option information to the selected data type to designate a data type corresponding to the new workout type, and wherein the selected data type may include at least one recommended measurement element for acquiring the workout measurement information.

According to an embodiment, based on a measurement element for location tracking being designated to the selected data type, the at least one processor may be configured to: control the display module to display a map reflecting a distance value measured in accordance with execution of the new workout type and location information.

According to an embodiment, the at least one processor may be configured to control the display module to display a workout log list providing workout measurement information of the new workout type and workout measurement information for at least one other workout type, and wherein the workout log list is separately displayed for each workout type or each workout duration.

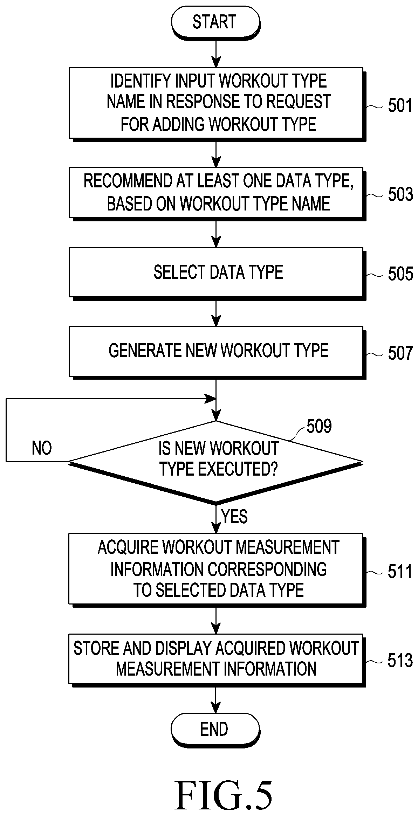

FIG. 5 illustrates an example of a method of operating electronic devices according to an embodiment. Each of a first electronic device (for example, the first electronic device 101 of FIG. 1, 3, or 4B) and a second electronic device (for example, the second electronic device 201 of FIG. 2, 3, or 4A) according to an embodiment may perform operations as illustrated in FIG. 5. Hereinafter, for convenience of description, the first electronic device is described by way of example of the operation method illustrated in FIG. 5. Since the second electronic device can perform the same operation method illustrated in FIG. 5, a description of the operation method of the second electronic device made with reference to FIG. 5 is omitted.

According to an embodiment, the first electronic device may perform operations for executing a workout application, displaying at least one workout type which can be provided by the executed workout application, generating new workout types in the executed workout application, and acquiring a result according to the execution of the new workout types.

Referring to FIG. 5, the first electronic device according to an embodiment may identify an input workout type name in response to a request for adding the workout type in operation 501. For example, the workout type name may be input through a user's utterance or an input window displayed on the execution screen of the workout application. For example, the workout type name may be selected from a list including recommended workout type names displayed on the execution screen.

In operation 503, the first electronic device may recommend at least one data type based on the workout type name. The first electronic device may store in advance mapping information between workout type names and at least one data type in a memory of the first electronic device (for example, the memory 130 of the electronic device 101 of FIG. 1). Further, the first electronic device may search for the workout type name through an external device, a server, the Internet, or the like and acquire information on similar workout type names, relevant workout types, or relevant data types. For example, the first electronic device may compare the input workout type name with preset workout type names included in mapping information, determine a workout type name having the highest similarity, and select the workout type name having the highest similarity as the recommended data type. The first electronic device may determine, as the recommended data type, a data type having the highest correlation with the input workout type name among at least one data type on the basis of information acquired from the outside. The electronic device may determine, as the recommended data type, a type having the highest correlation with the input workout type name through an artificial intelligence module within the first electronic device. The first electronic device may recommend a data type on the basis of recent workout measurement information of the user as well as the similarity or the correlation with the input workout type name. The first electronic device may display and recommend a plurality of data types having the correlation according to a correlation priority. At least one data type may be a condition for measuring workout execution of a new workout type to be generated in accordance with the workout type name. At least one data type is a measurement element and may include at least one of a distance, a speed, pace, calories, a heart rate, workout measurement duration, the number of repetitive workouts, cadence, or a workout set. In addition, the data type may further include another element which can be measured as the measurement element when the workout type is executed. When at least one data type is recommended, the first electronic device may display objects for selecting at least one recommended data type, guidance information, or objects for additional options (for example, important data or workout intensity selection) on the display module.

In operation 505, the first electronic device may identify a data type for acquiring workout measurement information selected by the user or automatically from among at least one recommended data type. The first electronic device may designate a data type including additional options (for example, important data or workout intensity selection) selected in accordance with the selected data type.

In operation 507, the first electronic device may generate a new workout type for acquiring workout measurement information corresponding to the selected data type and store information related to the new workout type. The information related to the generated new workout type may include identification information (for example, a type name or an identification code) of the workout type, an icon (for example, an image or a graphic element) indicating the workout type, information on the selected data type, or additional information (for example, a priority or memo information of the user). The first electronic device may add objects (for example, at least one of images, icons, graphic elements, or text) indicating the generated new workout type to the workout type list which can be provided and display the workout type list on the display module 160.

In operation 509, the first electronic device may identify whether the new workout type is executed. Operation 511 may be performed when the new workout type is executed on the basis of the identification result, and operation 509 may be performed again when the new workout type is not executed. When at least one of workout types which can be provided other than the new workout type is being executed during operations 501 to 509, workout measurement information according to execution of at least one other workout type may be acquired and stored.

In operation 511, the first electronic device may acquire workout measurement information corresponding to the selected data type. The first electronic device may acquire measurement data measured by at least one sensor included in a sensor module (for example, the sensor module 176 of FIG. 1) or a GPS module (not shown) for each of at least one measurement element designated to the selected data type. The first electronic device may acquire workout measurement information configured on the basis of measurement data measured for each of at least one measurement element.

In operation 513, the first electronic device may store the acquired workout measurement information in a memory (for example, the memory 130 of FIG. 1) and display the same on a display module (for example, the display module 160 of FIG. 1). The first electronic device may acquire workout result information according to the workout for a predetermined period on the basis of the workout measurement information and display the acquired workout result information on the display module.

According to an embodiment, the first electronic device may transmit information related to the new workout type, workout measurement information of the new workout type, or additional information (for example, at least one piece of weather information, user information, previous workout result information, workout measurement information of another workout type, or user input information) to the second electronic device 201 or another external electronic device through a communication module (for example, the communication module 190 of FIG. 1). The first electronic device may be synchronized with the second electronic device to perform operations of the executed workout application and measure and share workout measurement information. The second electronic device may transmit information related to the new workout type added by the second electronic device, workout measurement information acquired by the second electronic device, or additional information to the first electronic device 101 or another external electronic device through a communication module (for example, the communication module 230 of FIG. 2) and perform operations for synchronization with the first electronic device 101.

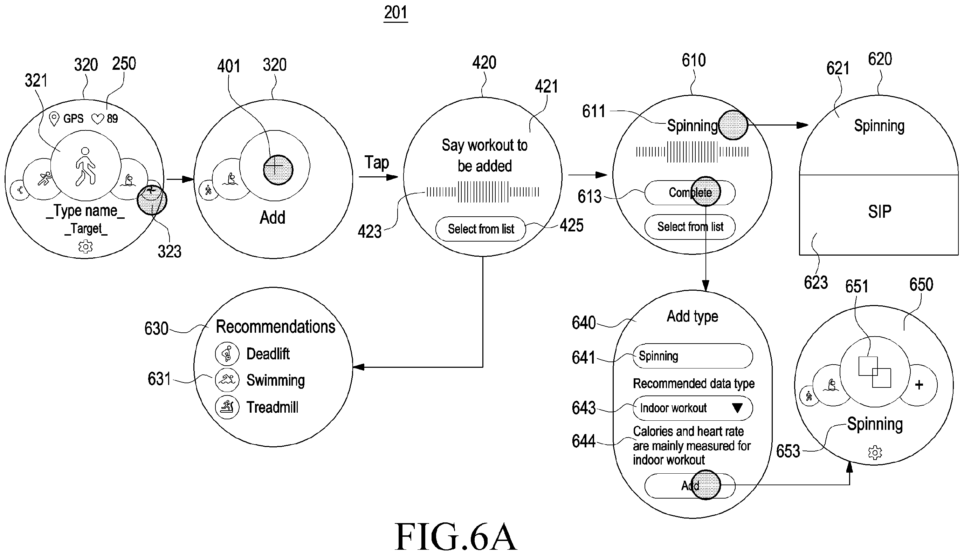

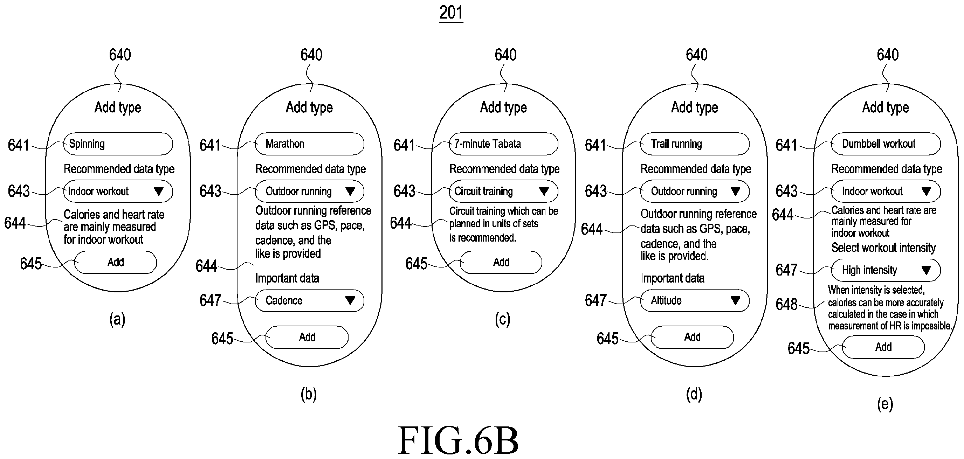

FIGs. 6A and 6B illustrate an example in which the second electronic device generates workout types according to an embodiment.

Referring to FIGs. 2 and 6A, the second electronic device 201 according to an embodiment may display at least one workout type 321 which can be provided by the executed workout application on the execution screen 320. When a selection input of the additional object 401 (for example, a menu, a button, an icon, an image, or a graphic element) displayed on the execution screen 320 is identified, the second electronic device 201 may display an execution screen 420 including an object 421 for inducing a voice input (for example, "Say a workout to be added"), an object 423 indicating detection of a voice input, and/or an object 425 for selecting a workout type to be directly selected by the user on the display module 250.

According to an embodiment, when voice information is input by a user's utterance, the second electronic device 201 may display a workout type name 611 (for example, spinning) on an execution screen 610. When it is required to modify text of the workout type name corresponding to voice information, the second electronic device 210 may display an execution screen 620 for modifying the type name on the display module 250. The second electronic device 201 may display the execution screen 620 including an object 621 indicating the type name and an object 623 for a user input for modifying text.

According to an embodiment, when a selection input of the object 425 for selecting the workout to be added is identified, the second electronic device 201 may display an execution screen 630 for recommending at least one workout type to be added on the display module 250. The second electronic device 210 may display objects (for example, a recommended list 631 (for example, the recommended list 413 of FIG. 4A) including an icon image and a workout type name) on the execution screen 630. When the workout type to be added to the recommended list 413 is selected, the second electronic device 201 may display a workout name 611 of the selected workout type on the execution screen 610.

According to an embodiment, when a completion object 613 displayed on the execution screen 610 is selected, the second electronic device 201 may identify a workout type corresponding to the workout type name 611 as a new workout type (for example, spinning) to be added and display an execution screen 640 for designating a data type as a condition for measuring measurement data when the new workout type is executed. The second electronic device 201 may display an object 641 (for example, a type name or an icon) indicating a new workout type, an object 643 for selecting at least one recommended data type (for example, indoor workout), guidance information 644 of data to be measured, and an additional object 645 on the execution screen 640. When some objects included in the execution screen 640 escape the display area of the display module 250 and thus not all the objects are displayed, the second electronic device 201 may display some escaped objects in the display area of the display module 250 in response to an input of a predetermined gesture (for example, swipe or scroll).

According to an embodiment, the second electronic device 201 may generate a new workout type (for example, spinning) on the basis of the data type 643 (for example, indoor workout) selected from at least one recommended data type. The second electronic device 201 may store the generated new workout type in the memory 240 and display an object (for example, an icon, an image, or a graphic element) 651 indicating the generated new workout type and a type name 653 of the generated new workout type on the execution screen 650. The processor 210 may transmit information related to the new workout type to the first electronic device 101 through the communication module 230 and perform synchronization. The information related to the generated new workout type may include identification information (for example, a type name or an identification code) of the workout type, an icon (for example, an image or a graphic element) indicating the workout type, information on the selected data type, or additional information (for example, a priority or memo information of the user).

Referring to FIGs. 2 and 6B, when a new workout type, which is to be added, corresponding to the type name input into the object 641 indicating the type name on the execution screen 640 is, for example, spinning, the second electronic device 201 according to an embodiment may recommend a data 643 as the indoor workout on the basis of spinning as illustrated in FIG. 6B(a). For example, indoor workouts which are the data type corresponding to spinning may include at least one measurement element among calories, a heart rate, or workout hours, and further include other measurement elements which can be measured during indoor workouts. The second electronic device 201 may display guidance information 644 (for example, "calories and a heart rate are mainly measured for indoor workouts.") of the measurement element included in the data type for the indoor workout corresponding to spinning.

According to an embodiment, as illustrated in FIG. 6B(b), when the new workout type, which is to be added, corresponding to the type name input into the object 641 indicating the type name on the execution screen 640 is, for example, marathon, the second electronic device 201 may recommend a data type 643 as an outdoor workout on the basis of marathon. For example, outdoor workouts which are the data type corresponding to marathon may include at least one measurement element among a GPS, pace, cadence, a distance, a speed, calories, or a heart rate and may further include other measurement elements which can be measured during outdoor workouts. The second electronic device 201 may display guidance information 644 (for example, "Outdoor running reference data such as a GPS, pace, cadence, and the like is provided.") of the measurement element included in the data for the outdoor workout corresponding to marathon. The second electronic device 201 may designate one of at least one measurement element included in the data type as important data (for example, cadence) through an additional option 647 or may designate a measurement element selected according to a user input from among at least one measurement element included in the data type as important data. The measurement element corresponding to the designated important data may be highlighted and displayed on the execution screen of new workout types in the second electronic device.

According to an embodiment, when the new workout type, which is to be added, corresponding to the type name input into the object 641 indicating the type name on the execution screen 640 is, for example, Tabata (for example, Tabata for 7 minutes), the second electronic device 201 may recommend the data type 643 corresponding to Tabata as circuit training as illustrated in FIG. 6B(c). For example, circuit training which is the data type corresponding to Tabata may include at least one measurement element among the number of repetitive workouts, a set, workout hours, or biometric information and may further include other measurement elements which can be measured during circuit training. The second electronic device 201 may display guidance information 644 (for example, "Circuit training which can be planned in units of sets is recommended.") of the measurement element included in the data type for the indoor workout corresponding to Tabata.

According to an embodiment, as illustrated in FIG. 6B(d), when the new workout type, which is to be added, corresponding to the type name input into the object 641 indicating the type name on the execution screen 640 is, for example, trail running, the second electronic device 201 may recommend the data type 643 as outdoor running on the basis of trail running. For example, outdoor running which is the data type corresponding to trail running may include at least one measurement element among a GPS, pace, cadence, a distance, a speed, calories, or a heart rate and may further include other measurement elements which can be measured during outdoor running. The second electronic device 201 may display guidance information 644 (for example, "outdoor running reference data such as a GPS, pace, cadence, and the like is provided.") of the measurement element included in the data type for outdoor running corresponding to trail running. The second electronic device may designate one of at least one measurement element included in the data type as important data through an additional option 647 or designate a measurement element selected according to a user input from among at least one measurement element included in the data type as important data (for example, altitude). The measurement element corresponding to the designated important data may be highlighted and displayed on the execution screen of new workout types in the second electronic device.

According to an embodiment, when the new workout type, which is to be added, corresponding to the type name input into the object 641 indicating the type name on the execution screen 640 is, for example, a dumbbell workout, the second electronic device 201 may recommend the data type 643 as the indoor workout on the basis of the dumbbell workout as illustrated in FIG. 6B(e). For example, indoor workouts which are the data type corresponding to the dumbbell workout may include at least one measurement element among calories, a heart rate, workout hours, or the number of repetitive workouts and may further include other measurement elements which can be measured during the outdoor workout. The second electronic device 201 may display guidance information 644 (for example, "Calories and a heart rate are mainly measured for the indoor workout.") of the measurement element included in the data type for the indoor workout corresponding to the dumbbell workout. The second electronic device 201 may choose workout intensity selection (for example, high intensity) through the additional option 647. The second electronic device 201 may display guidance information 648 (for example, when intensity is selected, calories can be more accurately calculated if measurement of a heart rate (HR) is not possible.") of the additional option 647.

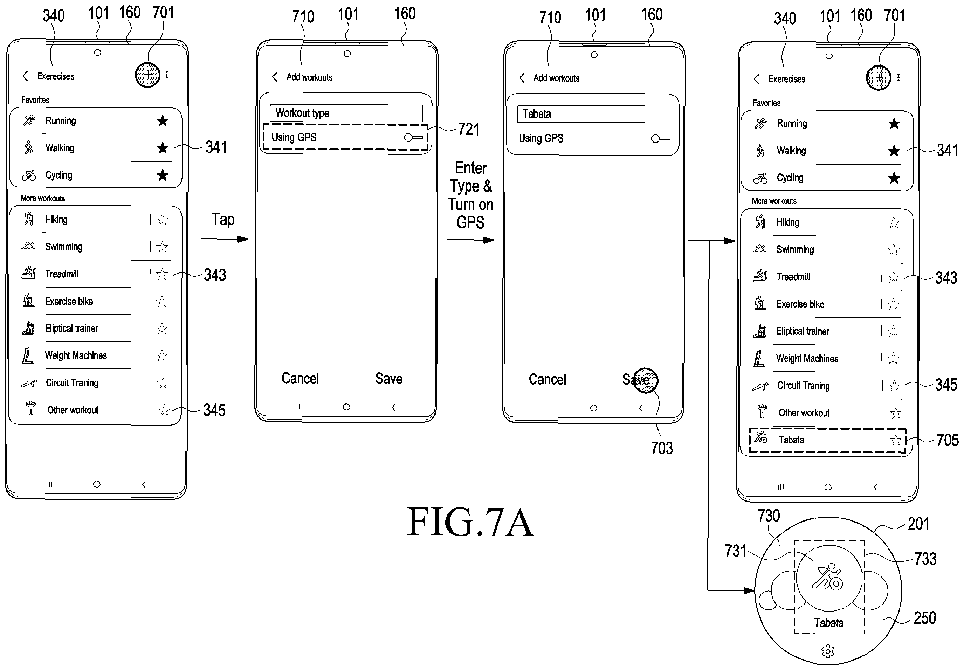

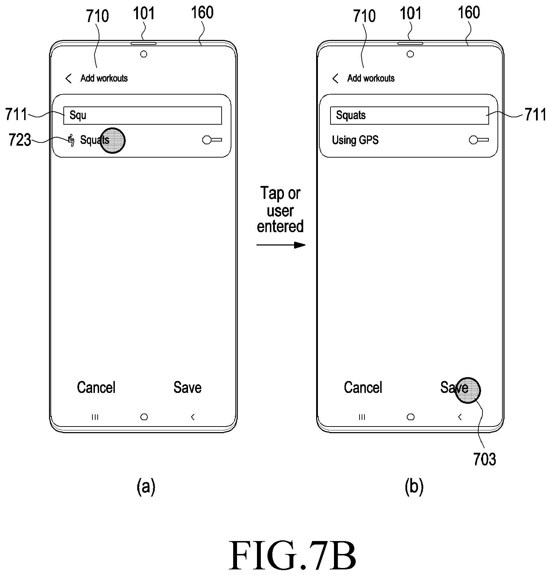

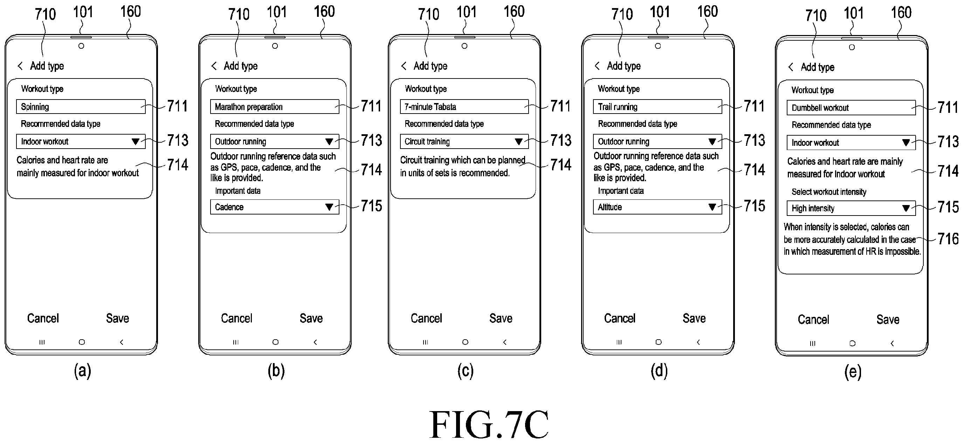

FIGs. 7A, 7B, and 7C illustrate an example in which the first electronic device adds workout types according to an embodiment.

Referring to FIG. 7A, the first electronic device 101 according to an embodiment may display the list 345 including objects indicating workout types 341 and 343 which can be provided by the executed workout application on the execution screen 340. When a selection input of an additional object 701 (for example, a menu, a button, an icon, an image, or a graphic element) displayed on the execution screen 250 is identified, the first electronic device 101 may display an execution screen 710 (for example, the execution screen 440 of FIG. 4) for adding a new workout type on the display module 160. The execution screen 710 may include an object (or an input window) 711 for receiving an input type name and an object 721 for selecting whether to use a GPS for the new workout type corresponding to the input type name. When the type name (for example, Tabata) is input, the first electronic device 101 may generate a new workout type (for example, Tabata) corresponding to the type name according to an input of a save object 703 displayed on the execution screen 710, add the generated new workout type 705 to the list 345 displayed on the execution screen 340, and display the list. The first electronic device 101 may transmit information related to the generated new workout type 705 to the second electronic device 201. The second electronic device 201 may display an execution screen 730 including an object 731 (for example, an icon, an image, or a graphic element) corresponding to the new workout type 705 and a type name 733 on the display module 250 on the basis of the received information related to the new workout type 705.

Referring to FIG. 7B, when text (for example, "squ") for inputting the type name into the input object 711 (or the input window) of the execution screen 710 is input, the first electronic device 101 according to an embodiment may automatically complete and display text 723 (for example, "squats") corresponding to the type name.

Referring to FIG. 7C, when the new workout type, which is to be added, corresponding to the type name input into the object 711 indicating the type name on the execution screen 710 is, for example, spinning, the first electronic device 101 according to an embodiment may recommend the data type 713 as the indoor workout on the basis of spinning as illustrated in FIG. 7C(a). For example, indoor workouts which are the data type corresponding to spinning may include at least one measurement element among calories, a heart rate, or workout hours, and further include other measurement elements which can be measured during indoor workouts. The first electronic device 101 may display guidance information 714 (for example, "calories and a heart rate are mainly measured for indoor workouts.") of the measurement element included in the data type for the indoor workout corresponding to spinning.

According to an embodiment, as illustrated in FIG. 7C(b), when the new workout type, which is to be added, corresponding to the type name input into the object 711 indicating the type name on the execution screen 710 is, for example, marathon, the first electronic device 101 may recommend a data type 713 as an outdoor workout on the basis of marathon. For example, outdoor workouts which are the data type corresponding to marathon may include at least one measurement element among a GPS, pace, cadence (for example, the time required between steps), a distance, a speed, calories, or a heart rate and may further include other measurement elements which can be measured during outdoor workouts. The first electronic device 101 may display guidance information 714 (for example, "Outdoor running reference data such as a GPS, pace, cadence, and the like is provided.") of the measurement element included in the data for the outdoor workout corresponding to marathon. The first electronic device 101 may designate one of at least one measurement element included in the data type as important data (for example, cadence) through the additional option 715 or designate a measurement element selected according to a user input from among at least one measurement element included in the data type as important data. The measurement element corresponding to the designated important data may be highlighted and displayed on the execution screen of new workout types in the second electronic device.

According to an embodiment, when the new workout type, which is to be added, corresponding to the type name input into the object 711 indicating the type name on the execution screen 710 is, for example, Tabata (for example, 7-minute Tabata), the first electronic device 101 may recommend the data type 713 corresponding to Tabata as circuit training as illustrated in FIG. 7C(c). For example, circuit training which is the data type corresponding to Tabata may include at least one measurement element among the number of repetitive workouts, a set, workout hours, or biometric information and may further include other measurement elements which can be measured during circuit training. The first electronic device 101 may display guidance information 714 (for example, "Circuit training which can be planned in units of sets is recommended.") of the measurement element included in the data type for the indoor workout corresponding to Tabata.

According to an embodiment, as illustrated in FIG. 7B(d), when the new workout type, which is to be added, corresponding to the type name input into the object 711 indicating the type name on the execution screen 710 is, for example, trail running, the first electronic device 101 may recommend the data type 713 as outdoor running on the basis of trail running. For example, outdoor running which is the data type corresponding to trail running may include at least one measurement element among a GPS, pace, cadence, a distance, a speed, calories, or a heart rate and may further include other measurement elements which can be measured during outdoor running. The first electronic device 101 may display guidance information 714 (for example, "Outdoor running reference data such as a GPS, pace, cadence, and the like is provided.") of the measurement element included in the data type for outdoor running corresponding to trail running. The first electronic device 101 may designate one of at least one measurement element included in the data type as important data through the additional option 715 or designate a measurement element selected according to a user input from among at least one measurement element included in the data type as important data (for example, altitude). The measurement element corresponding to the designated important data may be highlighted and displayed on the execution screen of new workout types in the second electronic device.

According to an embodiment, when the new workout type, which is to be added, corresponding to the type name input into the object 711 indicating the type name on the execution screen 710 is, for example, a dumbbell workout, the first electronic device 101 may recommend the data type 713 as the indoor workout on the basis of the dumbbell workout as illustrated in FIG. 7B(e). For example, indoor workouts which are the data type corresponding to the dumbbell workout may include at least one measurement element among calories, a heart rate, workout hours, or the number of repetitive workouts and may further include other measurement elements which can be measured during the outdoor workout. The first electronic device 101 may display guidance information 714 (for example, "Calories and a heart rate are mainly measured for the indoor workout.") of the measurement element included in the data type for the indoor workout corresponding to the dumbbell workout. The first electronic device 101 may select workout intensity (for example, high intensity) through the additional option 715. The first electronic device 101 may display guidance information 716 (for example, "When intensity is selected, calories can be more accurately calculated if measurement of a heart rate (HR) is not possible.") of the additional option 715.

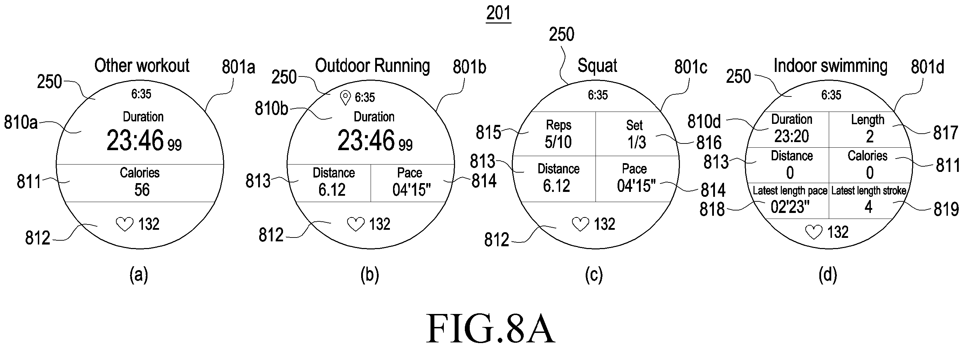

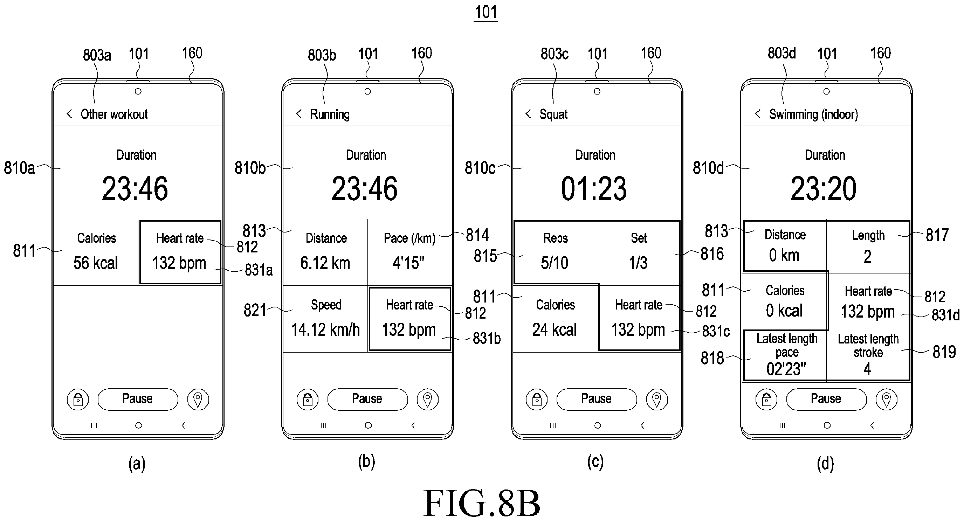

FIG. 8A illustrates an example of a workout measurement result by the second electronic device according to an embodiment, and FIG. 8B illustrates an example of a workout measurement result by the first electronic device according to an embodiment.

Referring to FIG. 8A, according to an embodiment, when a generated new workout type is executed, the second electronic device 201 may acquire measurement data according to performance of a workout by the user for a new workout type on the basis of at least one measurement element included in a data type of the new workout type. For example, all or at least some of the measurement data may be acquired through at least one sensor included in a sensor module (for example, the sensor module 220 of FIG. 2) of the second electronic device 201 or/and a GPS module (not shown). In another example, all or at least some of the measurement data may be received from the first electronic device (for example, the electronic device 101 of FIG. 1). The second electronic device 201 may display workout measurement information including measurement data acquired on the basis of at least one measurement element on the display module 250. The second electronic device 201 may be synchronized with the first electronic device and may transmit workout measurement information to the first electronic device to display workout measurement information.

According to an embodiment, as illustrated in FIG. 8A(a), when the input type name is other workout, the second electronic device 201 may measure measurement elements included in the data type selected in accordance with other workout and acquire measurement data for each measurement element. For example, the second electronic device 210 may display an execution screen 801a including calories 811 (for example, calories 56) and a heart rate 812 (for example, 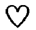 132) on the display module 250 as measurement data acquired for workout measurement duration 810a in accordance with other workout. For example, the second electronic device 210 may further display a current time (for example, 6:35).

According to an embodiment, as illustrated in FIG. 8A(b), when the input type name is outdoor running, the second electronic device 201 may measure measurement elements included in the data type selected in accordance with outdoor running and acquire measurement data for each measurement element. For example, the second electronic device 210 may display an execution screen 801b including a heart rate 812 (for example, 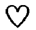 132), a distance 813 (for example, distance 6.12), and pace 814 (pace 4' 15") on the display module 250 as measurement data acquired for workout measurement duration 810b (For example, duration 23:46) in accordance with outdoor running. For example, the second electronic device 210 may further display an object indicating GPS information and a current time (for example, 6:35).

According to an embodiment, as illustrated in FIG. 8A(c), the second electronic device 201 may measure measurement elements included in the data type selected in accordance with squats and acquire measurement data for each measurement elements. For example, the second electronic device 210 may display an execution screen 801c including calories 811 (for example, 24), a heart rate 812 (for example, 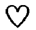 132), a distance 813 (for example, distance 6.12), pace 814 pace 4' 15"), the number of repetitive sets 815 (for example, reps 5/10), and a set 816 (for example, set 1/3) on the display module 250 as measurement data acquired for workout measurement duration 801c (for example, 6:35). For example, the second electronic device 210 may further display a current time (for example, 6:35).

According to an embodiment, as illustrated in FIG. 8A(d), the second electronic device 201 may measure measurement elements included in the data type selected in accordance with indoor swimming and acquire measurement data for each measurement element. For example, the second electronic device 210 may display an execution screen 801d including calories 811 (for example, calories 0), a heart rate 812 (for example, 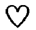 132), a distance 813 (for example, distance 0), a length 817 (for example, length 2), latest length pace 818 (for example, latest length pace 02' 23"), and a latest length stroke 819 (for example, latest length stroke 4) on the display module 250 as measurement data acquired for workout measurement duration 810d (for example, duration 23:20). For example, the second electronic device 210 may further display a current time (for example, 6:35).

According to an embodiment, when displaying measurement elements illustrated in FIGs. 8A(a) to 8A(d) on the display module 250 of the second electronic device 201, the second electronic device 201 may highlight and display a measurement element corresponding to important data designated when a new workout type is generated on the execution screen of the new workout type. For example, compared to other measurement element(s), the measurement element corresponding to important data may be displayed with different display location, size, color, brightness, blinking, and the like. Further, display orders, locations, sizes, or visual effects of measurement elements displayed on the display module 250 may be changed or designated through a configuration function or a user interface provided in the second electronic device 201.

Referring to FIG. 8B, according to an embodiment, when the generated new workout type is executed, the first electronic device 101 may acquire measurement data according to performance of the workout by the user for the new workout type on the basis of at least one measurement element included in the data type of the new workout type. For example, all or at least some of the measurement data may be acquired through at least one sensor included in a sensor module (for example, the sensor module 176 of FIG. 1) of the first electronic device 101 or/and a GPS module (not shown). In another example, all or at least some of the measurement data may be received from the second electronic device (for example, the electronic device 201 of FIG. 2). The first electronic device 101 may display workout measurement information including measurement data acquired on the basis of at least one measurement element on the display module 160. The first electronic device 101 may be synchronized with the second electronic device and may transmit workout measurement information to the second electronic device to display workout measurement information.

According to an embodiment, as illustrated in FIG. 8B(a), when the input type name is other workouts, the first electronic device 101 may measure measurement elements included in the data type selected in accordance with other workouts and acquire measurement data for each measurement element. For example, the first electronic device 101 may display an execution screen 803a including calories 811 (for example, calories 56 kcal) and a heart rate 812 (for example, heart rate 132 bpm) on the display module 160 as measurement data acquired for workout measurement duration 810a (duration 23:46) in accordance with other workouts.

According to an embodiment, as illustrated in FIG. 8B(b), when the input type name is outdoor running, the first electronic device 101 may measure measurement elements included in the data type selected in accordance with outdoor running and acquire measurement data for each measurement element. For example, the first electronic device 101 may display an execution screen 803b including a heart rate 812 (for example, heart rate 132 bpm), a distance 813 (for example, distance 6.12 km), pace 814 (pace(/km) 4' 15"), and a speed 821 (for example, speed 14.12 km/h) on the display module 160 as measurement data acquired for workout measurement duration 810b (for example, duration 23:46) in accordance with outdoor running.

According to an embodiment, as illustrated in FIG. 8B(c), when the input type name is squat, the first electronic device 101 may measure measurement elements included in the data type selected in accordance with squat and acquire measurement data for each measurement element. For example, the first electronic device 101 may display an execution screen 803c including calories 811 (for example, calories 24), a heart rate 812 (for example, heart rate 132 bpm), the number of repetitive sets 815 (for example, reps 5/10), and a set 816 (for example, set 1/3) on the display module 160 as measurement data acquired for workout measurement duration 810c (duration 01:23).

According to an embodiment, as illustrated in FIG. 8B(d), when the input type name is indoor swimming, the first electronic device 101 may measure measurement elements included in the data type selected in accordance with indoor swimming and acquire measurement data for each measurement element. For example, the first electronic device 101 may display an execution screen 803d including calories 811 (for example, calories 0 kcal), a heart rate 812 (for example, heart rate 132 bpm), a distance 813 (for example, distance 0 km), a length 817 (for example, length 2), latest length pace 818 (for example, latest length pace 02' 23"), and a latest length stroke 819 (for example, latest length stroke 4) on the display module 250 as measurement data acquired for workout measurement duration 810d (for example, duration 23:20) in accordance with indoor swimming.

As illustrated in FIGs. 8B(a) to 8B(d), box marking 831a, 831b, 831c, and 831d on the execution screens 803a to 803d may indicate data measured by the second electronic device 201 in the state in which the user wears the second electronic device. When the user is not wearing the second electronic device 201, the measurement data separated by the box marking 831a, 831b, 831c, and 831d may not be displayed.

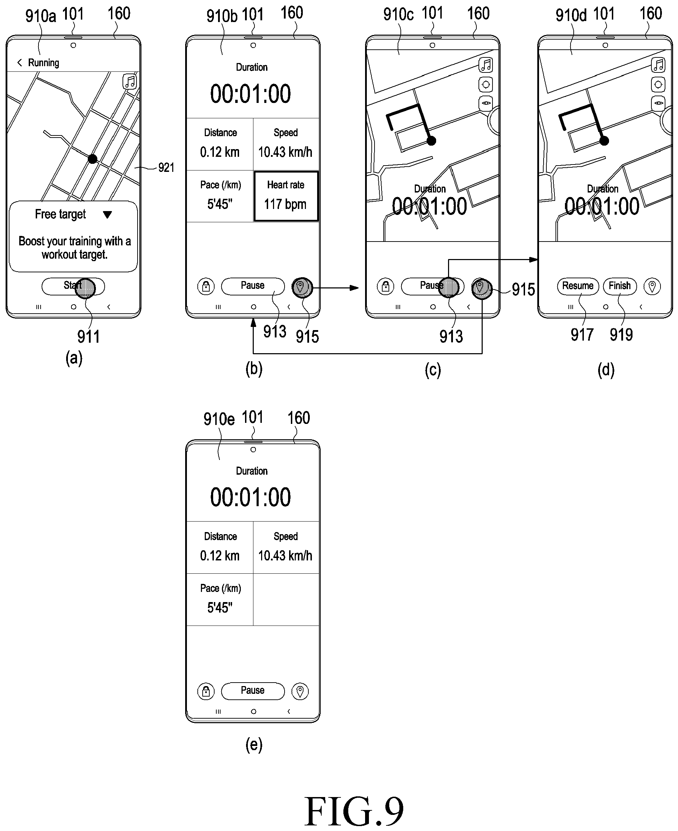

FIG. 9 illustrates an example of a workout measurement result and an example of an execution result of a new workout type in the first electronic device according to an embodiment.

Referring to FIG. 9, according to an embodiment, when a new workout type generated in accordance with an input type name is, for example, indoor running, the first electronic device 101 may identify the location of the user based on GPS information and display a map 921 showing the identified location on an execution screen 910a for displaying workout measurement information as illustrated in FIG. 9A. The first electronic device 101 may display additional option selection (for example, free target) for outdoor running and guidance information (for example, "boost your training with a workout target") on the execution screen 910a.

According to an embodiment, when a start object 911 displayed on the execution screen 910a is input, the first electronic device 101 may identify whether a communication connection is made with the second electronic device and/or whether a heart rate is measured. When the connection with the second electronic device (for example, the second electronic device 201 of FIG. 2) is made and measurement of the heart rate is not needed on the basis of the identification result, an execution screen 910b including measurement data (for example, distance 0.12km, speed 10.43km/h, pace 5'45", and heart rate 117 bpm) measured in accordance with outdoor running may be displayed as illustrated in FIG. 9(b). When a pause object 913 displayed on the execution screen 910b is input, the first electronic device may display a movement path on the map 921 on the basis of measurement data of the distance as illustrated in FIG. 9(d). When an object 915 for showing a map displayed on the execution screen 910b is input, the first electronic device 101 may display an execution screen 910c as illustrated in FIG. 9(c) showing the map 921 displaying the movement path based on measurement data of the distance. When the pause object 913 displayed on the execution screen 910c is input, the first electronic device 101 may display an execution screen 910d as illustrated in FIG. 9(d).

According to an embodiment, when the connection with the second electronic device is not made or measurement of the heart rate is not needed, the first electronic device 101 may display an execution screen 910e including measurement data (for example, distance 0.12km, speed 10.43km/h, and pace 5'5") of measurement elements which can be measured by the first electronic device on the display module 160 except for measurement data (for example, heart rate 117 bpm) of measurement elements which can be measured by the second electronic device among the measurement elements included in the data type in accordance with outdoor running as illustrated in FIG. 9(e).

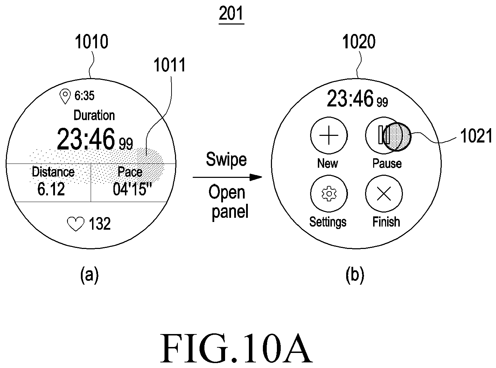

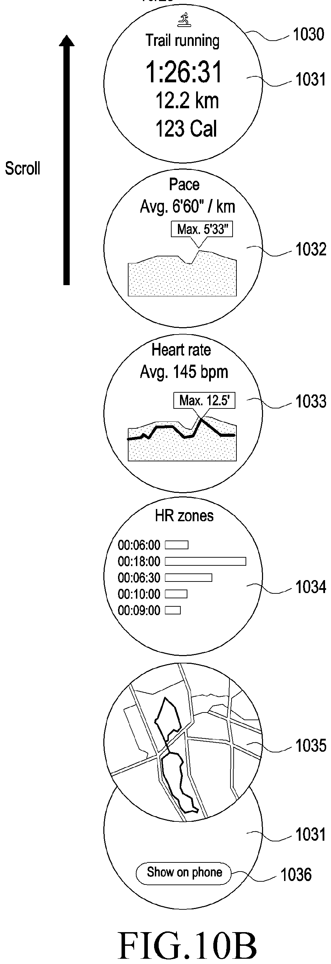

FIGs. 10A and 10B illustrate an example of an execution result of a new workout type in the second electronic device according to various embodiments.

Referring to FIGs. 10A and 10B, when a predetermined gesture 1011 (for example, swipe, tap, or scroll) is input (for example, open panel request) on an execution screen 1010 (for example, at least one of the execution screens 801a to 801d of FIG. 8A) for displaying workout measurement information as illustrated in FIG. 10A, the second electronic device 201 according to an embodiment may switch to an execution screen 1020 for selecting functions related to workout execution. When an input of a pause object 1021 is identified on the execution screen 1020, the second electronic device 201 may pause an operation for measuring measurement data corresponding to the selected data type as illustrated in FIG. 10A(b). The second electronic device 201 may acquire workout measurement information including measurement data measured for a workout measurement period and display the acquired workout measurement information (at least one of 1031, 1032, 1033, 1034, or 1035) in a display area 1030 of the display module 250 as illustrated in FIG. 10B. When all of the workout measurement information (at least one of 1031, 1032, 1033, 1034, or 1035) are not displayed in the display area 1030 of the display module 250, the second electronic device 201 may scroll in a predetermined direction and display information, which is not displayed, in the display area 1030. For example, when a type name is trail running, the second electronic device 201 may display workout measurement information (at least one of 1031, 1032, 1033, 1034, or 1035) in the display area 1030 of the display module 250 in accordance with measurement elements (for example, at least one of calories, a distance, pace, a heart rate, or GPS information) included in a data type of trail running. For example, the second electronic device 201 may display workout measurement duration for example, 1:26:31), an object (for example, 12.2 km) for a movement distance, and an object (for example, 123 cal) for calories in the display area 1030 of the display module 250 as workout result information 1031 to be preferentially displayed and, when a scroll in an up direction is input, display, for example, objects (for example, avg.6'60 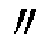 /km and a graph) for pace in the display area 1030 as the following workout measurement information 1032. When an additional scroll in an up direction is input in the state in which the workout measurement information 1032 is displayed, the second electronic device 201 may display, for example, objects (for example, avg. 145 bpm and a graph) for a heart rate in the display area 1030 as the following workout measurement information 1033. When an additional scroll in an up direction is input in the state in which the workout measurement information 1033 is displayed, the second electronic device 201 may display, for example, objects (for example, graphs showing a heart rate for each time zone) for heart rate zones in the display area 1030 as the following workout measurement information 1034. When an additional scroll in an up direction is input in the state in which the workout measurement information 1034 is displayed, the second electronic device 201 may display, for example, a map 1035 showing a movement path based on GPS information in the display area 1030 as the following workout measurement information 1035. When an additional scroll in an up direction is made and there is no workout measurement information to be displayed in the state in which the workout measurement information 1035 is displayed, the second electronic device 201 may share workout measurement information with the first electronic device 101 and display an object 1036 (for example, a button, a function, or a menu) making a request for displaying in the first electronic device 101 in the display area 1030.

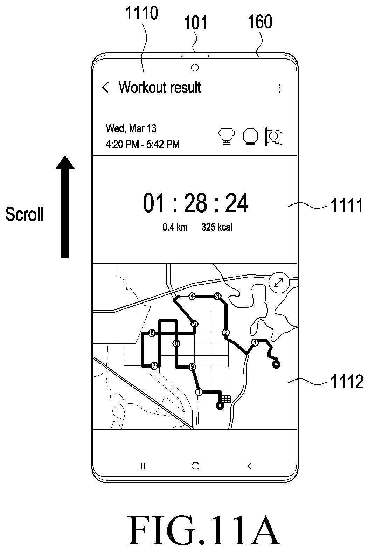

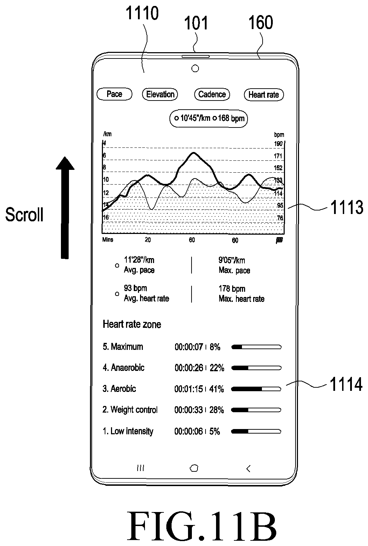

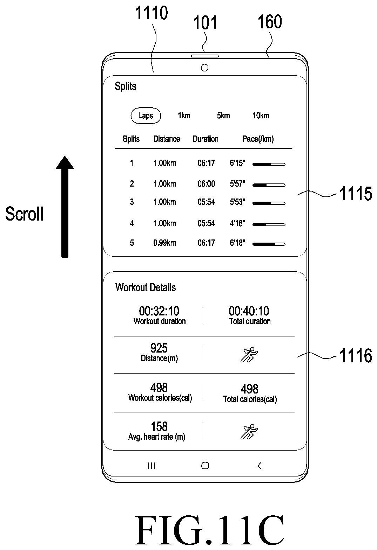

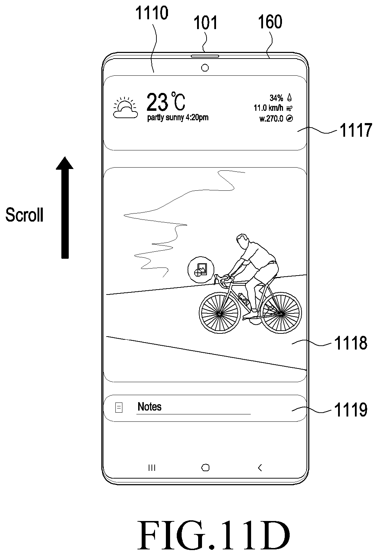

FIGs. 11A, 11B, 11C, and 11D illustrate an example of an execution result of a new workout type in the first electronic device according to various embodiments.

Referring to FIGs. 11A to 11D, the first electronic device 101 according to an embodiment may acquire workout output information measured by a sensor module (for example, the sensor module 176 of FIG. 1) or a GPS module or workout measurement information received from the second electronic device 201 and display the acquired workout measurement information on the display module 160. When all of the acquired workout measurement information are not displayed in a display area 1110 of the display module 160, the first electronic device 101 may scroll in a predetermined direction and display information, which is not displayed, in the display area 1110. For example, when a type name is trail running, the first electronic device 101 may display workout measurement information (at least one of 1111, 1112, 1113, 1114, 1115, 1116, or 1117) in the display area 1110 of the display module 250 in accordance with measurement elements (for example, at least one of calories, a distance, pace, a heart rate, or GPS information) included in a data type of trail running.

According to an embodiment, as illustrated in FIG. 11A, the first electronic device 101 may display, in the display area 1110 of the display module 160, objects indicating workout measurement duration (for example, 1:28:24), a movement distance (for example, 0.4 km), and calories (for example, 325 kcal) as workout measurement information 1111 to be preferentially displayed, and a map showing a movement distance based on GPS information as workout measurement information 1112.

According to an embodiment, when a scroll in an up direction is input, the first electronic device 101 may display the following workout measurement information 1113 and 1114 in the display area 1110 of the display module 160. For example, the first electronic device 101 may display objects (for example, an average pace value (11'28 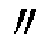 /km), a maximum pace value (9'05 " /km), and a graph) for pace and a heart rate (for example, an average heart rate value (93 bpm), a maximum heart rate value (178 bpm), and a graph) in the display area 1110 as the following workout measurement information 1113. For example, the first electronic device 101 may display objects (for example, graphs showing a heart rate for each time zone) for heart rate zones in the display area 1110 as the following workout measurement information 1114.

According to an embodiment, when an additional scroll in an up direction is input in the state in which the workout measurement information 1113 and 1114 are displayed, the first electronic device 101 may display measurement information 1115 for splits and detailed workout measurement information (workout details) 1116 in the display area 1110 of the display module 160 as illustrated in FIG. 11C. For example, the first electronic device 101 may display measurement data for each split (for example, a distance value, duration, and a pace value) in the display area 1110 as the measurement information 1115 for splits. With respect to the measurement information 1115 for splits, measurement data for each split may be displayed according to a round trip section (laps) or each predetermined zone (for example, 1 km, 5 km, or 10 km). For example, the first electronic device 101 may display workout duration, total workout duration (total duration), a distance value (distance(m)), a calorie value (workout calories(cal)), a total calorie value (total calories(cal)), and an average heart rate (avg heart rate(m)) in the display area 1110 as the detailed workout measurement information (workout details) 1116.

According to an embodiment, when an additional scroll in an up direction is input in the state in which the measurement information for splits 1115 and the detailed workout measurement information (workout details) 1116 are displayed, the first electronic device 101 may display at least one of weather information 1117 during the workout, a workout-related image 1118, or a user memo 1119 as illustrated in FIG. 11D. An object (for example, a button, a function, or a menu) (not shown) making a request for displaying in the first electronic device 101 may be displayed by sharing workout measurement information with the first electronic device 101.

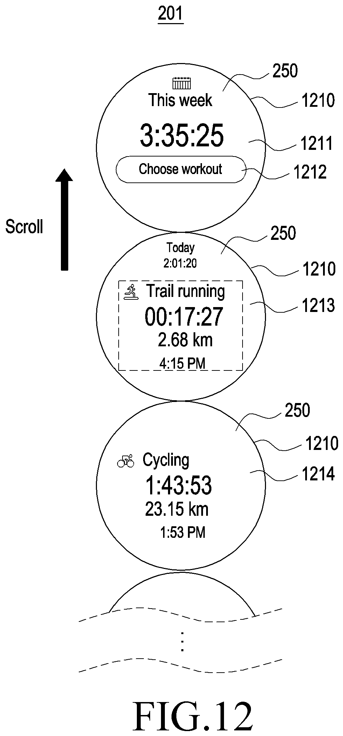

FIG. 12 illustrates an example of an execution result of a new workout type in the second electronic device according to various embodiments.

Referring to FIG. 12, the second electronic device 201 according to an embodiment may display workout results of workouts performed by the user for a predetermined period or a period selected by the user in the form of a workout log list. For example, the second electronic device 201 may display total workout hours 1211 (for example, 3:35:25) by workout types performed for this week and an object 1212 (for example, choose workout) for selecting a workout type in a display area 1210 of the display module 250. When at least one workout type is selected through the object 1212 for selecting the workout type, the second electronic device 201 may display workout measurement information 1213 (for example, trail running, 00:17:27, 2.68km, and 4:45pm) which is the workout result for the selected workout type (for example, trail running or cycling) or workout measurement information 1214 (for example, cycling, 1:43:53, 23.15km, and 1:53pm) in the display area 1210.

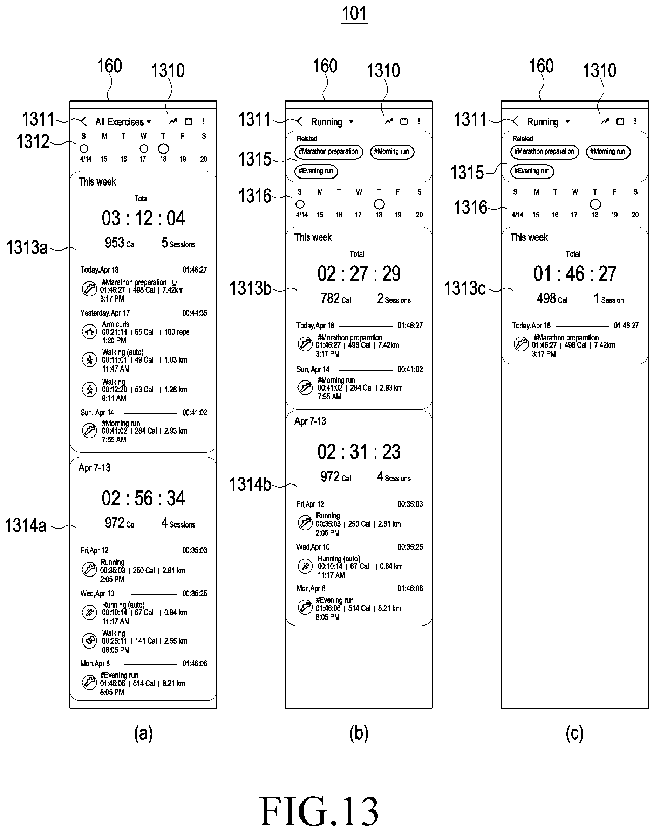

FIG. 13 illustrates an example of a workout measurement result in the first electronic device according to various embodiments.

Referring to FIG. 13, the first electronic device 101 according to an embodiment may display the workout results of workouts performed by the user for a predetermined period or a period selected by the user in the form of a workout log list. For example, the first electronic device 101 may display an object 1311 for selecting a workout type, an object 1312 (for example, calendar) for selecting workout duration, and workout measurement information 1313a, 1313b, or 1313c including total workout hours by workout types performed for the selected duration, total measurement data measured for total measurement hours, and measurement data of performed workout types in a display area 1310 of the display module 160 in the form a list. When at least some pieces of the workout measurement information 1313a, 1313b, or 1313c are not displayed in the display area 1310 of the display module 160, the first electronic device 101 may display at least some pieces of the information which are not displayed in the display area 1310 by scrolling in a predetermined direction. When an additional scroll is input in the state in which the measurement information 1313a, 1313b, or 1313c is displayed, the first electronic device 101 may display a week before predetermined duration and workout measurement information 1314a or 1314b of workout types performed on a selected particular date or on the day in the display area 1310 of the display module 160. For example, when all of the performed workout types are selected, the first electronic device 101 may display workout measurement information 1313a or 1314a for all of the workout types in the display area 1310 of the display module 160. When the selected workout type is, for example, running, the first electronic device 101 may display workout measurement information 1313b or 1314b corresponding to running. The first electronic device 101 may display objects 1315 indicating detailed workout types related to running (for example, at least one of marathon, marathon preparation, morning run, or evening run) in the display area 1310 of the display module 160. When one of the objects 1315 indicating the detailed workout types is selected, the first electronic device 101 may display workout measurement information 1313c for a predetermined period (for example, this week) with respect to the selected detailed type 1316 (for example, marathon or marathon preparation) in the display area 1310 of the display module 160.

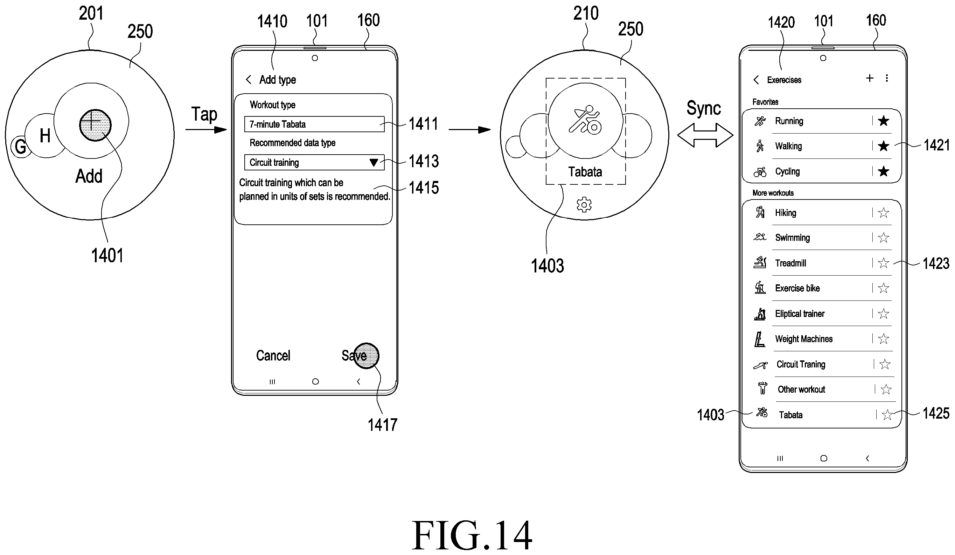

FIG. 14 illustrates an example of a method of an operation for adding a new workout type in the first electronic device and the second electronic device according to an embodiment.

Referring to FIG. 14, the second electronic device 201 according to an embodiment may execute a workout application and display at least one workout type which can be provided through the executed workout application. When one of the at least one displayed workout type is selected, the second electronic device 201 may display a workout result (or workout measurement information) according to execution of the selected workout type.

According to an embodiment, the second electronic device 201 may identify whether there is a request for adding a new workout type. When there is the request for adding the new workout type on the basis of the identification result, the second electronic device 201 may transmit a message making the request for adding the new workout type to the first electronic device 101. For example, when an object 1401 (for example, an image, an icon, or a graphic element) indicating addition of the new workout type is selected, the second electronic device 201 may transmit the message making the request for adding the new workout type to the first electronic device 101.

According to an embodiment, the first electronic device 101 may receive a message making the request for adding the new workout type, execute a workout application, and acquire a workout type name (for example, 7-minute Tabata) for the workout type to be additionally provided by the workout application. The type name may be acquired (or identified) on the basis of text information directly input by the user through an input window 1411 displayed on the execution screen 1410 of the workout application or identification information of the workout type selected from recommended workout types.

According to an embodiment, the first electronic device 101 may recommend at least one data type identified on the basis of the acquired type name and select a data type to be applied to the workout type to be added from among at least one recommended data type. For example, when the acquired type name is 7-minute Tabata, the first electronic device 101 may recommend a data type 1413 (for example, circuit training) corresponding to 7-minute Tabata and display guidance information 1415 (for example, "Circuit training which can be planned in units of sets is recommended.") for the recommended data type on the display module 160.

According to an embodiment, the first electronic device 101 may generate a new workout type corresponding to the workout type name on the basis of the selected data type. For example, when an input of selecting a save object 1417 is identified, the first electronic device 101 may generate a new workout type (Tabata) 1403 corresponding to the workout type name of 7-minute Tabata on the basis of the selected data type 1413 (for example, circuit training). The first electronic device 101 may add the generated new workout type 1403 to a list 1425 including the workout types 1421 and 1423, which can be provided, displayed on the execution screen 1420.

According to an embodiment, the first electronic device 101 may transmit information related to the generated new workout type (for example, Tabata 1403) to the second electronic device 201. According to an embodiment, the second electronic device 201 may store the received information related to the new workout type 1403 and display the same on the display module 250. The new workout type 1403 may be displayed by an icon (or an image) and text indicating a type name.

According to an embodiment, the first electronic device 101 may identify execution of the generated new workout type 1403 and acquire measurement data measured on the basis of all or at least some of the measurement elements included in the selected data type 1413 while the user executes the new workout type 1403. The second electronic device 101 may be synchronized with the first electronic device 101 and may simultaneously or individually acquire measurement data measured on the basis of all or at least some of the measurement elements included in the selected data type.

According to an embodiment, the first electronic device 101 and the second electronic device 201 may be synchronized with each other and may transmit the acquired measurement data to share the same. The first electronic device 101 and the second electronic device 201 may acquire workout measurement information on the basis of measurement data and display the acquired workout measurement information.

According to an embodiment, in the operation methods described with reference to FIGs. 5 and 14, each of the first electronic device and the second electronic device may recommend at least one workout type to be added and/or at least one data type on the basis of at least one of a workout habit of the user, body (or physical strength) information, biometric information, age, information related to an environment (or context) (time, weather, season, or place), or previous workout measurement information. When displaying acquired workout measurement information according to execution of a new workout type, each of the first electronic device and the second electronic device may provide at least one of information related to a workout to be reinforced, workout types which are effective when the new workout type is performed together, an image related to the new workout type, information related to exercise equipment or clothes for the new workout type, information related to recommended foods or drug for the new workout type, or information related to foods or drug that should be avoided for the new workout type.

According to an embodiment, a method of operating an electronic device (for example, the electronic device 101 of FIGs. 1 and 2) may include an operation of acquiring a workout type name in response to a request for adding a workout type, recommending at least one data type, based on the workout type name, an operation of generating a new workout type corresponding to the workout type name, based on a data type selected from among the at least one recommended data type, and an operation of acquiring workout measurement information corresponding to the selected data type in response to execution of the new workout type.

According to an embodiment, the method may further include an operation of displaying the acquired workout measurement information on a display module of the electronic device.

According to an embodiment, the operation of displaying the acquired workout measurement information on the display module of the electronic device may include displaying, when a measurement element for location tracking is designated to the selected data type, a map reflecting a distance value measured in accordance with execution of the new workout type and location information.

According to an embodiment, the method may further include an operation of transmitting information related to a new workout type including the workout type name and the selected data type to an external electronic device through a communication module of the electronic device.

According to an embodiment, the method may further include an operation of displaying a list including recommended workout type on a display module of the electronic device in response to the request for adding the workout type; and an operation of transmitting the list including the recommended workout types to an external electronic device.

According to an embodiment, the operation of acquiring the workout measurement information may include an operation of acquiring measurement data corresponding to at least one measurement element included in the selected data type through at least one sensor included in a sensor module of the electronic device and an operation of acquiring the workout measurement information, based on the acquired measurement data.

According to an embodiment, the operation of acquiring the workout measurement information may include an operation of performing synchronization with an external electronic device, an operation of acquiring measured measurement data corresponding to at least one measurement element included in the selected data type from the external electronic device, and an operation of acquiring the workout measurement information, based on the acquired measurement data.

According to an embodiment, the operation of generating the new workout type may include an operation of providing additional option information for the selected data type, an operation of selecting the provided additional option information, based on a predetermined priority, and an operation of adding the selected additional option information to the selected data type to designate a data type corresponding to the new workout type, and wherein the selected data type may include at least one recommended measurement element for acquiring the workout measurement information.

According to an embodiment, the method may further include an operation of displaying a workout log list providing workout measurement information of the new workout type and workout measurement information for at least one other workout type on a display module of the electronic device. The workout log list may be separately displayed for each workout type or each workout duration.

According to an embodiment, in a non-transitory computer-readable storage medium storing one or more programs, the one or more programs comprising instructions configured to, when executed by at least one processor of an electronic device, cause the electronic device to perform an operation of acquiring a workout type name in response to a request for adding a workout type, an operation of recommending at least one data type, based on the workout type name, an operation of generating a new workout type corresponding to the workout type name, based on a data type selected from among the at least one recommended data type, and an operation of acquiring workout measurement information corresponding to the selected data type in response to execution of the new workout type.

Embodiments disclosed in this document are presented for explanation and understanding of the disclosed technology, and do not limit the scope of the technology disclosed in this document. Accordingly, the scope of this document should be construed to include all modifications based on the technical idea of this document or various other embodiments.

The electronic device according to various embodiments disclosed in this document may be various types of electronic devices. The electronic device may include, for example, a portable communication device (for example, a smart phone), a computer device, a portable multimedia device, a portable medical device, a camera, a wearable device, or a home appliance. The electronic device according to an embodiment of this document is not limited to the above-described devices.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B", "at least one of A and B", "at least one of A or B", "A, B, or C", "at least one of A, B, and C", and "at least one of A, B, or C", may include any one of, or all possible combinations of the items enumerated together in a corresponding one of the phrases. As used herein, such terms as "1st" and "2nd", or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with", "coupled to", "connected with", or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic", "logic block", "part", or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStoreTM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components or operations may be omitted, or one or more other components or operations may be added. Alternatively or additionally, a plurality of components (e.g., modules or programs) may be integrated into a single component. In such a case, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device comprising:
a memory; and
at least one processor connected to the memory,
wherein the at least one processor is configured to:
acquire a workout type name in response to a request for adding a workout type,
recommend at least one data type, based on the workout type name,
generate a new workout type corresponding to the workout type name, based on a data type selected from among the at least one recommended data type, and
acquire workout measurement information corresponding to the selected data type in response to execution of the new workout type.

2. The electronic device of claim 1, further comprising:
a display module comprising a display connected to the at least one processor, and
a communication module comprising communication circuitry connected to the at least one processor,
wherein the at least one processor is configured to:
control the display module to display a list including recommended workout types in response to the request for adding the workout type,
control the communication module to transmit the list including the recommended workout types to the external electronic device,
control the display module to display the acquired workout measurement information, and
control the communication module to transmit information related to the new workout type including the workout type name and the selected data type to an external electronic device, and
wherein the communication module is configured to communicate with the external electronic device through short-range wireless communication.

3. The electronic device of claim 1 or claim 2, further comprising:
a sensor module comprising at least one sensor connected to the at least one processor,
wherein the at least one processor is configured to:
acquire measurement data corresponding to at least one measurement element included in the selected data type through at least one sensor included in the sensor module, and
acquire the workout measurement information, based on the acquired measurement data

4. The electronic device of any one of claim 1 to claim 3, wherein the at least one processor is configured to perform synchronization with an external electronic device, acquire measured measurement data corresponding to at least one measurement element included in the selected data type from the external electronic device, and acquire the workout measurement information, based on the acquired measurement data.

5. The electronic device of any one of claim 1 to claim 4, wherein at least one processor is configured to:
provide additional option information for the selected data type,
select the provided additional option information, based on a specified priority, and
add the selected additional option information to the selected data type to designate a data type corresponding to the new workout type, and
wherein the selected data type includes at least one recommended measurement element for acquiring the workout measurement information.

6. The electronic device of any one of claim 1 to claim 5, wherein, based on a measurement element for location tracking being designated to the selected data type, the at least one processor is configured to control the display module to display a map reflecting a distance value measured in accordance with execution of the new workout type and location information.

7. The electronic device of any one of claim 1 to claim 6, wherein the at least one processor is configured to control the display module to display a workout log list providing workout measurement information of the new workout type and workout measurement information for at least one other workout type, and
wherein the workout log list is separately displayed for each workout type or each workout duration.

8. A method of operating an electronic device, the method comprising:
acquiring a workout type name in response to a request for adding a workout type;
recommending at least one data type, based on the workout type name;
generating a new workout type corresponding to the workout type name, based on a data type selected from among the at least one recommended data type; and
acquiring workout measurement information corresponding to the selected data type in response to execution of the new workout type.

9. The method of claim 8, further comprising displaying the acquired workout measurement information on a display module of the electronic device,
wherein the displaying of the acquired workout measurement information on the display module of the electronic device comprises: displaying, based on a measurement element for location tracking being designated to the selected data type, a map reflecting a distance value measured in accordance with execution of the new workout type and location information.

10. The method of claim 8 or claim 9, further comprising:
transmitting information related to a new workout type including the workout type name and the selected data type to an external electronic device through a communication module of the electronic device; displaying a list including a recommended workout type on a display module of the electronic device in response to the request for adding the workout type; and
transmitting the list including the recommended workout types to an external electronic device.

11. The method of any one of claim 8 to claim 10, wherein the acquiring of the workout measurement information comprises:
acquiring measurement data corresponding to at least one measurement element included in the selected data type through at least one sensor included in a sensor module of the electronic device; and
acquiring the workout measurement information, based on the acquired measurement data.

12. The method of any one of claim 8 to claim 11, wherein the acquiring of the workout measurement information further comprises:
performing synchronization with an external electronic device;
acquiring measured measurement data corresponding to at least one measurement element included in the selected data type from the external electronic device; and
acquiring the workout measurement information, based on the acquired measurement data.

13. The method of any one of claim 8 to claim 12, wherein the generating of the new workout type comprises:
providing additional option information for the selected data type;
selecting the provided additional option information based on a specified priority; and
adding the selected additional option information to the selected data type to designate a data type corresponding to the new workout type; and
wherein the selected data type includes at least one recommended measurement element for acquiring the workout measurement information.

14. The method of any one of claim 8 to claim 13, further comprising displaying a workout log list providing workout measurement information of the new workout type and workout measurement information for at least one other workout type on a display module of the electronic device, and
wherein the workout log list is separately displayed for each workout type or each workout duration.

15. A non-transitory computer-readable storage medium storing one or more programs, the one or more programs comprising instructions configured to, when executed by at least one processor of an electronic device, cause the electronic device to:
acquiring a workout type name in response to a request for adding a workout type;
recommending at least one data type, based on the workout type name;
generating a new workout type corresponding to the workout type name, based on a data type selected from among the at least one recommended data type; and
acquiring workout measurement information corresponding to the selected data type in response to execution of the new workout type.
